# EUROPEAN PATENT SPECIFICATION

(11) **EP 0 813 868 B1**
(45) Date of publication and mention of the grant of the patent: **01.06.2005**
(21) Application number: 97105352.5
(22) Date of filing: 18.11.1991
(51) Int. Cl.: A61K 31/17, A61K 31/47, A61K 31/44, A61K 31/535, A61K 31/505, A61K 31/34, A61K 31/415, A61K 31/495, A61K 31/215, A61K 31/18, A61K 31/325, A61K 31/40, A61K 31/195

(54) **Retroviral protease inhibitors**
Retrovirusprotease Inhibitoren
Inhibiteurs de protéases rétrovirales

(30) Priority: 19.11.1990 US 615210
(43) Date of publication of application: 29.12.1997
(62) Divisional of application: 92901068.4
(73) Proprietor: MONSANTO COMPANY, St. Louis, Missouri 63167 (US); G.D. Searle & Co., Skokie, Illinois 60077 (US)
(72) Inventor: Clare, Michael, Skokie, Illinois 60077 (US); Freskos, John Nicholas, Clayton, Missouri 63105 (US); Heintz, Robert Martin, Ballwin, Missouri 63021 (US); Decrescenzo, Gary Anthony, St. Peters, Missouri 63376 (US); Getman, Daniel Paul, St. Louis, Missouri 63146 (US); Lin, Ko-Chung, St. Louis, Missouri 63146 (US); Mueller, Richard August, Glencoe, Illinois 60022 (US); Talley, John Jeffrey, Chesterfield, Missouri 63017 (US); Sun, Eric Tak On, Buffalo Grove, IL 60039 (US); Reed, Kathryn Lea, Richmond Heights, Missouri 63117 (US); Vasquez, Michael Lawrence, Gurnee, Illinois 60031 (US)
(74) Representative: Colens, Alain

(56) References cited:
- EP-A- 0 159 156
- WO-A-89/10920
- FR-A- 2 620 451

## Description

### BACKGROUND OF THE INVENTION

### 1. Field of the Invention

The present invention relates to retroviral protease inhibitors and, more particularly, relates to novel compounds and a composition and method for inhibiting retroviral proteases. This invention, in particular, relates to urea-containing hydroxyethylamine protease inhibitor compounds, a composition and method for inhibiting retroviral proteases such as human immunodeficiency virus (HIV) protease and for treating a retroviral infection, e.g., an HIV infection. The subject invention also relates to processes for making such compounds as well as to intermediates useful in such processes.

### 2. Related Art

During the replication cycle of retroviruses, gag and gag-pol gene products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. It has also been shown through site-directed mutagenesis of an aspartic acid residue in the HIV protease that processing of the gag precursor protein is prevented. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit replication of structural proteins and, more importantly, the retroviral protease itself. In this manner, retroviral replication proteases can be effectively inhibited.

Several classes of mimetic compounds have been proposed, particularly for inhibition of proteases, such as for inhibition of HIV protease. Such mimetics include hydroxyethylamine isosteres and reduced amide isosteres. See, for example, EP O 346 847; EP O 342,541; Roberts et al, "Rational Design of Peptide-Based Proteinase Inhibitors, "Science, 248, 358 (1990); and Erickson et al, "Design Activity, and 2.8Å Crystal Structure of a C₂ Symmetric Inhibitor Complexed to HIV-1 Protease," Science, 249, 527 (1990).

Several classes of mimetic compounds are known to be useful as inhibitors of the proteolytic enzyme renin. See, for example, U.S. No. 4,599,198; U.K. 2,184,730; G.B. 2,209,752; EP O 264 795; G.B. 2,200,115 and U.S. SIR H725. Of these, G.B. 2,200,115, GB 2,209,752, EP O 264,795, U.S. SIR H725 and U.S. 4,599,198 disclose urea-containing hydroxyethylamine renin inhibitors. However, it is known that, although renin and HIV proteases are both classified as aspartyl proteases, compounds which are effective renin inhibitors generally cannot be predicted to be effective HIV protease inhibitors.

### BRIEF DESCRIPTION OF THE INVENTION

The present invention is directed to virus inhibiting compounds and compositions. More particularly, the present invention is directed to retroviral protease inhibiting compounds and compositions, to a method of inhibiting retroviral proteases, to processes for preparing the compounds and to intermediates useful in such processes. The subject compounds are characterized as urea-containing hydroxyethylamine inhibitor compounds.

### DETAILED DESCRIPTION OF THE INVENTION

In accordance with the present invention, there is provided the use of a compound represented by the formula: 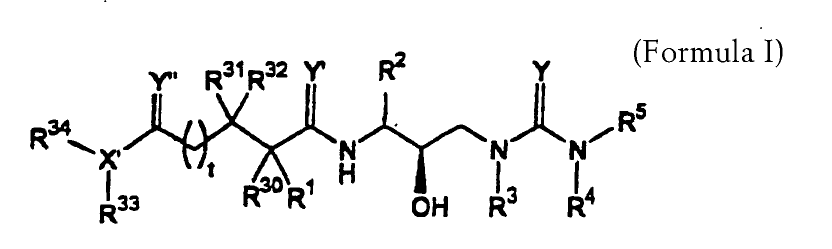 for preparing a medicament for inhibiting a retroviral protease, for treating a retroviral infection and for treating AIDS wherein:
R¹ represents hydrogen, -CH₂SO₂NH₂, alkyl and cycloalkyl radicals and amino acid side chains selected from the group consisting of asparagine, S-methyl cysteine and the corresponding sulfoxide and sulfone derivatives thereof, glycine, allo-isoleucine, alanine, leucine, tert-leucine, phenylalanine, ornithine, threonine, allo-threonine, isoleucine, histidine, norleucine, valine, glutamine, serine, aspartic acid and betacyano alanine side chains;
R² represents alkyl, aryl, cycloalkyl, cycloalkylalkyl and aralkyl radicals optionally substituted with a group selected from -OR⁹, -SR⁹, and halogen radicals wherein R⁹ represents hydrogen and alkyl radicals;
R³ represents alkyl, alkenyl, hydroxyalkyl, cycloalkyl, cycloalkylalkyl, heterocycloalkyl,
   heterocycloalkylalkyl, aryl, heteroaryl, aralkyl, and heteroaralkyl radicals;
R⁴ and R⁵ independently represent hydrogen and radicals as defined by R³, or R⁴ and R⁵, together with the nitrogen. atom to which they are bonded represent heterocycloalkyl and heteroaryl radicals;
R³⁰, R³¹ and R³² represent radicals as defined for R¹, or one of R¹ and R³⁰ together with one of R³¹ and R³² and the carbon atoms to which they are attached form a cycloalkyl radical;
R³³ and R³⁴ independently represent hydrogen and radicals as defined for R³, or R³³ and R³⁴ together with X' represent cycloalkyl, aryl, heterocyclyl and heteroaryl radicals, provided that when X' is O, R³⁴ is absent;
X' represents N, O or C(R¹⁷) wherein R¹⁷ represents hydrogen and alkyl radicals, Y, Y' and Y" independently represent O and S,
t represents 0 or 1; and
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro,
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

As utilized herein, the term "alkyl", alone or in combination, means a straight-chain or branched-chain alkyl radical containing from 1 to 10, preferably from 1 to 8, carbon atoms. Examples of such radicals include methyl, ethyl, n-propyl, isopropyl, n-butyl, isobutyl, sec-butyl, tert-butyl, pentyl, isoamyl, hexyl and octyl. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy and tert-butoxy. The term "cycloalkyl" means an alkyl radical which contains from about 3 to 8 carbon atoms and is cyclic. The term "cycloalkylalkyl" means an alkyl radical as defined above which is substituted by a cycloalkyl radical containing from about 3 to 8, preferably from about 3 to 6, carbon atoms. Examples of such cycloalkyl radicals include cyclopropyl, cyclobutyl, cyclopentyl and cyclohexyl. The term "aryl", alone or in combination, means a phenyl or naphthyl radical which optionally carries one or more substituents selected from alkyl, alkoxy, halogen, hydroxy, amino and nitro, such as phenyl, p-tolyl, 4-methoxyphenyl, 4-(tert-butoxy)phenyl, 4-fluorophenyl, 4-chlorophenyl, 4-hydroxyphenyl, 1-naphthyl and 2-naphthyl. The term "aralkyl", alone or in combination, means an alkyl radical as defined above in which one hydrogen atom is replaced by an aryl radical as defined above, such as benzyl and 2-phenylethyl. The term "aralkoxy carbonyl", alone or in combination, means a radical of the formula -C(O)-O-aralkyl in which the term "aralkyl" has the significance given above. An example of an aralkoxycarbonyl radical is benzyloxycarbonyl. The term "leaving group" generally refers to groups readily displaceable by a nucleophile, such as an amine, a thiol or an alcohol nucleophile. Such leaving groups are well known and include carboxylates, N-hydroxysuccinimide, N-hydroxybenzotriazole, halides, triflates, tosylates -OR and -SR. Preferred leaving groups are indicated herein where appropriate.

Procedures for preparing the compounds of Formula I are set forth below. It should be noted that the general procedure is shown as it relates to preparation of compounds having the preferred specified stereochemistry, namely where the stereochemistry about the hydroxyl group is designated as (R). However, such procedures are generally applicable to those compounds of opposite configuration, e.g., where the stereochemistry about the hydroxyl group is (S).

### Preparation of Compounds of Formula I

The compounds of the present invention represented by Formula I above can be prepared utilizing the following general procedure. An N-protected chloroketone derivative of an amino acid having the formula: 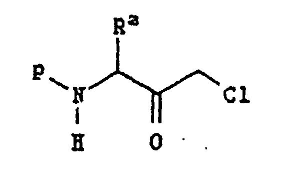 wherein P represents an amino protecting group, and R² is as defined above, is reduced to the corresponding alcohol utilizing an appropriate reducing agent. Suitable amino protecting groups are well known in the art and include carbobenzoxy, butyryl, t-butoxycarbonyl, acetyl, benzoyl and the like. A preferred amino protecting group is carbobenzoxy. A preferred N-protected chloroketone is N-benzyloxycarbonyl-L-phenylalanine chloromethyl ketone. A preferred reducing agent is sodium borohydride. The reduction reaction is conducted at a temperature of from -10°C to about 25°C, preferably at about 0°C, in a suitable solvent system such as, for example, tetrahydrofuran, and the like. The N-protected chloroketones are commercially available from Bachem, Inc., Torrance, California. Alternatively, the chloroketones can be prepared by the procedure set forth in S. J. Fittkau, J. Prakt. Chem., 315, 1037 (1973), and subsequently N-protected utilizing procedures which are well known in the art.

The resulting alcohol is then reacted, preferably at room temperature, with a suitable base in a suitable solvent system to produce an N-protected amino epoxide of the formula: 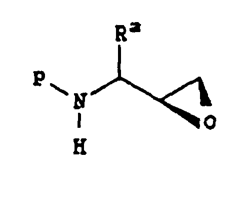 wherein P and R² are as defined above. Suitable solvent systems for preparing the amino epoxide include ethanol, methanol, isopropanol, tetrahydrofuran, dioxane, and the like including mixtures thereof. Suitable bases for producing the epoxide from the reduced chloroketone include potassium hydroxide, sodium hydroxide, potassium t-butoxide, DBU and the like. A preferred base is potassium hydroxide.

The amino epoxide is then reacted, in a suitable solvent system, with an equal amount, or preferably an excess of, a desired amine of the formula:

R³NH₂

wherein R³ is hydrogen or is as defined above. The reaction can be conducted over a wide range of temperatures, e.g., from about 10°C to about 100°C, but is preferably, but not necessarily, conducted at a temperature at which the solvent begins to reflux. Suitable solvent systems include those wherein the solvent is an alcohol, such as methanol, ethanol, isopropanol, and the like, ethers such as tetrahydrofuran, dioxane and the like, and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine, naphthylene methyl amine and the like. The resulting product is a 3-(N-protected amino)-3-(R²)-1-(NHR³)-propan-2-ol derivative (hereinafter referred to as an amino alcohol) can be represented by the formula: 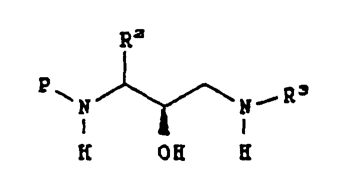 wherein P, R² and R³ are as described above.

For producing compounds of Formula I wherein R⁵ is hydrogen, the resulting amino alcohol described above is then reacted, in a suitable solvent system, with an isocyanate of the formula R⁴NCO wherein R⁴ is as defined above. Where Y in Formula I above is sulfur, the resulting amino alcohol is reacted with an isothiocyanate of the formula R⁴NCS under similar conditions. Suitable solvent systems include tetrahydrofuran, methylene chloride, and the like and mixtures thereof. The resulting product is a urea derivative of the amino alcohol or the corresponding sulfur analog thereof and can be represented by the formula: 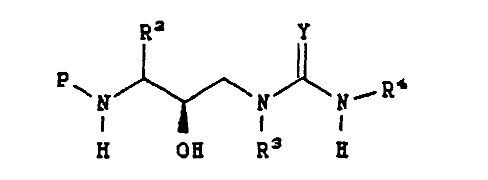 wherein P, Y, R², R³ and R⁴ are as defined above. The isocyanates of the formula R⁴NCO can be prepared by the reaction of an amine (R³NH₂) with phosgene, triphosgene, carbodiimidazole, or carbonate ((RO)₂CO) under conditions well-known in the art. The isothiocyanates of the formula R₄NCS can be prepared by similar procedures, e.g., reaction of the amine with thiophosgene, which are also well known in the art. In addition, the isocyanates and isothiocyanates are commercially available from Aldrich Chemical Company.

For preparing compounds of Formula I wherein R⁵ is other than hydrogen, the resulting amino alcohol described above is then reacted, in a suitable solvent system, with a compound represented by the formula: 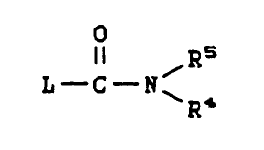 wherein R⁴ and R⁵ are as described above and L represents a leaving group such as a halide, e.g., chloride, imidazole radical, the radical p-NO₂-(C₆H₄)-O-, and the like. A preferred compound represented by this formula is a carbamoyl chloride. The corresponding sulfur analogs can be utilized where Y of Formula I is S.

The urea derivative of the amino alcohol and the corresponding sulfur analog can be represented by the formula:

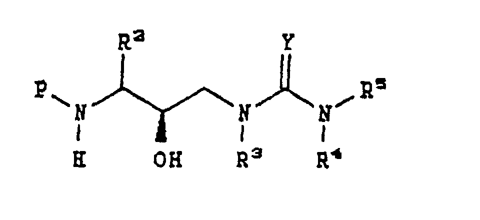

Following preparation of the urea derivative, or corresponding analogs wherein Y is S, the amino protecting group P is removed under conditions which will not affect the remaining portion of the molecule. These methods are well known in the art and include acid hydrolysis, hydrogenolysis and the like. A preferred method involves removal of the protecting group, e.g., removal of a carbobenzoxy group, by hydrogenolysis utilizing palladium on carbon in a suitable solvent system such as an alcohol, acetic acid, and the like or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative. Following neutralization of the salt, the amine is then further reacted with a diacid or anhydride prepared → 22 as follows.

To produce compounds of Formula I, starting with a lactate of the formula: 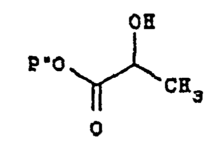 wherein P" represents alkyl radicals, such as, for example, ethyl, methyl, benzyl and the like. The hydroxyl group of the lactate is protected as its ketal by reaction in a suitable solvent system with methyl isopropenyl ether (1,2-methoxypropene) in the presence of a suitable acid. Suitable solvent systems include methylene chloride, tetrahydrofuran and the like as well as mixtures thereof. Suitable acids include POCl₃ and the like. It should be noted that well-known groups other than methyl isopropenyl ether can be utilized to form the ketal. The ketal is then reduced with diisobutylaluminum hydride (DIBAL) at -78°C to produce the corresponding aldehyde which is then treated with ethylidene triphenylphosphorane (Wittig reaction) to produce a compound represented by the formula:

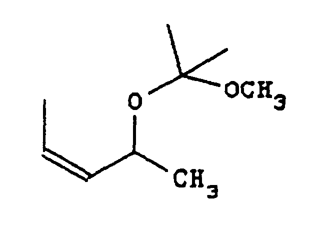

The ketal protecting group is then removed utilizing procedures well-known in the art such as by mild acid hydrolysis. The resulting compound is then esterified with isobutyryl chloride to produce a compound of the formula:

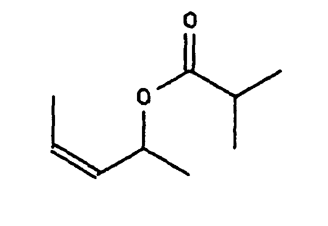

This compound is then treated with lithium diisopropyl amide at -78°C followed by warming of the reaction mixture to room temperature to effect a Claisen rearrangement ([3,3]) to produce the corresponding acid represented by the formula:

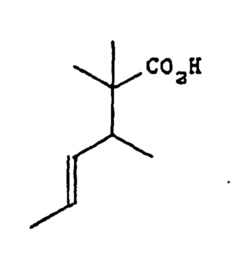

Treatment of the acid with benzyl bromide in the presence of a tertiary amine base, e.g., DBU, produces the corresponding ester which is then cleaved oxidatively to give a trisubstituted succinic acid:

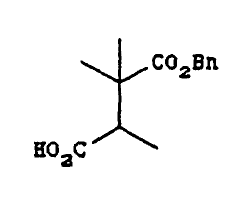

The trisubstituted succinic acid is then coupled to the urea isostere as described above. To produce the free acid, the benzyl ester is removed by hydrogenolysis to produce the corresponding acid. The acid can then be converted to the primary amide by methods well-known in the art.

An alternative method for preparing trisubstituted succinic acids involves reacting an ester of acetoacetic acid represented by the formula: 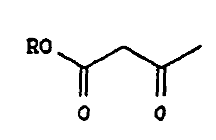 where R is a suitable protecting group, such as methyl, ethyl, benzyl or t-butyl with sodium hydride and a hydrocarbyl halide (R³¹X or R³²X) in a suitable solvent, e.g., THF, to produce the corresponding disubstituted derivative represented by the formula: 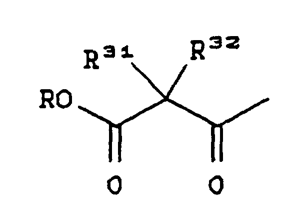 This disubstituted acetoacetic acid derivative is then treated with lithium diisopropyl amide at about -10°C and in the presence of PhN(triflate)₂ to produce a vinyl triflate of the formula:

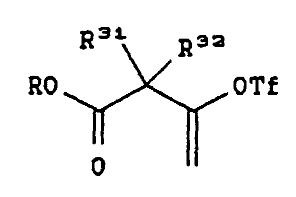

The vinyl triflate is then carbonylated utilizing a palladium catalyst, e.g., Pd₂(OAc)₂(Ph₃)P, in the presence of an alcohol (R"OH) or water (R"=H) and a base, e.g., triethylamine, in a suitable solvent such as DMF, to produce the olefinic ester or acid of the formula: 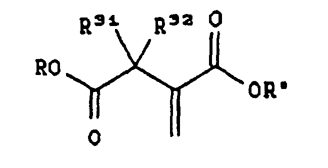 The olefin can then be subsequently asymmetrically hydrogenated, as described above, to produce a trisubstituted succinic acid derivative of the formula: 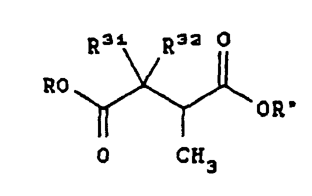 If R" is not H, R" can be removed by either hydrolysis, acidolysis, or hydrogenolysis, to afford the corresponding acid, which is then coupled to the urea isostere as described above and then, optionally, the R group removed to produce the corresponding acid, and optionally, converted to the amide.

Alternatively, one can react the urea isostere with either a suitably monoprotected succinic acid or glutaric acid of the following structure; 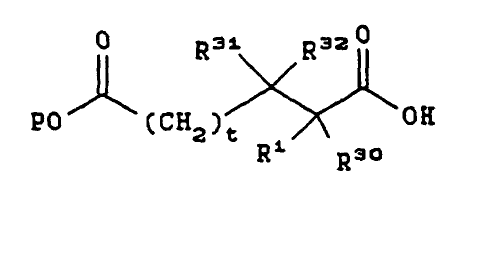 followed by removal of the protecting group and conversion of the resulting acid to an amide. One can also react an anhydride of the following structure; 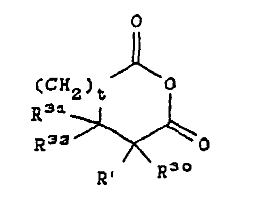 with the urea isostere and then separate any isomers or convert the resulting acid to an amide and then separate any isomers.

It is contemplated that where R³ of the amino alcohol intermediate is hydrogen, the inhibitor compounds can be prepared through reductive amination of the final product of the reaction between the amino alcohol and the amine or at any other stage of the synthesis for preparing the inhibitor compounds.

Contemplated equivalents of the general formulas set forth above for the antiviral compounds and derivatives as well as the intermediates are compounds otherwise corresponding thereto and having the same general properties wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

All reagents were used as received without purification. All proton and carbon NMR spectra were obtained on either a Varian VXR-300 or VXR-400 nuclear magnetic resonance spectrometer.

### Example 1

This example illustrates preparation of compounds of Formula I I wherein t is 1.

### 4-N-benzyl itaconamide.

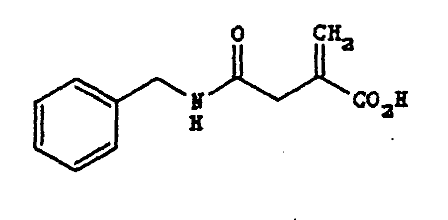

A 500 mL three necked round bottomed flask equipped with a dropping funnel, mechanical stirrer, nitrogen inlet and reflux condenser was charged with itaconic anhydride (33.6g, 0.3 mol) and 150 mL of toluene. This solution was added a solution of benzylamine (32.1g, 0.3 mol) in 50 mL of toluene dropwise over 30 m at room temperature. The solution was stirred at this temperature an additional 3h and then the solid product isolated by filtration on a Büchner funnel. The crude product, 64.6g 98%, was recrystallized from 300 mL of isopropyl alcohol to give after two crops 52.1g, 79% of pure product, mp 149-150 °C

### 2(R)-Methyl 4-N-benzyl succinamide.

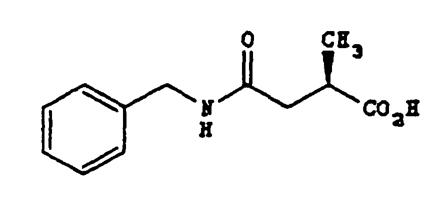

A large Fisher-Porter bottle was charged with the acid from the above reaction (10.95g, 0.05 mol), rhodium (R,R)-DiPAMP (220mg, 0.291 mmol) and 125 mL of degassed methanol. The solution was then hydrogenated at 40 psig for 16h at room temperature. After the hydrogen uptake ceased, the vessel was opened and the solution concentrated *in vacuo* to give a yellow solid, 11.05g, 100%. The product was then taken up in absolute ethanol and allowed to stand whereupon crystals of the desired product formed, 7.98g, 72%, mp 127-129 °C [a]_{D} @ 25 °C=+14.9° (c=1.332, EtOH), ¹H nmr (CDCl₃) 300MHz 7.30(m,5H), 6.80(brs, 1H), 4.41(d, J=5.8Hz, 2H), 2.94(m, 1H), 2.62(dd, J=8.1, 14.9Hz, 1H), 2.33(dd, J=5.5, 14.9Hz, 1H), 1.23(d, J=7.2Hz, 3H).

### 4-N(4-methoxybenzyl)itaconamide.

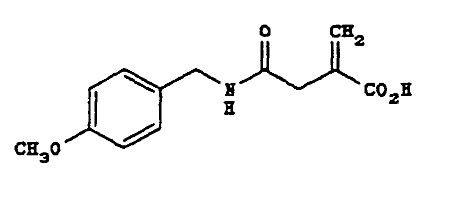

A 500 mL three necked round bottomed flask equipped with a dropping funnel, mechanical stirrer, nitrogen inlet and reflux condenser was charged with itaconic anhydride (44.8g, 0.4 mol) and 150 mL of toluene. This solution was added a solution of 4-methoxybenzylamine (54.8g, 0.4 mol) in 50 mL of toluene dropwise over 30 m at room temperature. The solution was stirred at this temperature an additional 2h and then the solid product isolated by filtration on a Büchner funnel. The crude product was recrystallized from ethyl acetate/ethanol to give after two crops 64.8g, 65% of pure product, mp 132-134 °C, ¹H nmr (CDCl₃) 300MHz 7.09(d, J=9.1Hz, 2H), 6.90(brt, J=5.9Hz, 1H), 6.74(d, J=9.1Hz, 2H), 6.22(s, 1H), 5.69(s, 1H), 4.24(d, J=5.9Hz, 2H), 3.69(s, 3H), 3.15(s, 2H). ¹³C nmr (CDCl₃) 170.52, 169.29, 159.24, 135.61, 131.08, 129.37, 128.97, 114.36, 55.72, 43.37, 40.58.

### 2(R)-Methyl 4-N(4-methoxybenzyl)succinamide

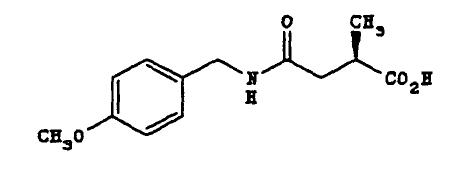

A large Fisher-Porter bottle was charged with the acid from the above reaction (5.00 g, 0.02 mol), rhodium (R,R)-DiPAMP (110 mg, 0.146 mmol) and 50 mL of degassed methanol. The starting acid was not completely soluble initially, but as the reaction progressed the solution became homogeneous. The solution was then hydrogenated at 40 psig for 16h at room temperature. After the hydrogen uptake ceased, the vessel was opened and the solution concentrated *in vacuo* to give a yellow solid. The crude product was then taken up in ethyl acetate and washed three times with sat. aq. NaHCO₃ solution. The combined aqueous extracts were acidified to pH=1 with 3 N HCl and then extracted three times with ethyl acetate. The combined ethyl acetate extracts were washed with brine, dried over anhyd. MgSO₄, filtered and concentrated to give the expected product as a white solid, 4.81g, 95%. This material was recrystallized from a mixture of methyl ethyl ketone/hexane to give 3.80g, 75% of pure product, [a]_{D} @ 25 °C=+11.6° (c=1.572, MeOH). ¹H nmr (CDCl₃) 300MHz 11.9(brs, 1H), 7.18(d, J=9.2Hz, 2H), 6.82(d, J=9.2Hz, 2H), 6.68(brt, J=5.6Hz, 1H), 4.33(d, J=5.6Hz, 2H), 3.77(s, 3H), 2.92(ddq, J=7.9, 5.4, 7.3Hz, 1H), 2.60(dd, J=5.4, 15.0Hz, 1H), 2.30(dd, J=7.9, 15.0Hz, 1H),1.22(d, J=7.3Hz, 3H).

### Butanediamide, N'-[3-[[[(1,1-dimethylethyl)amino]carbonyl](3-methylbutyl)amino]-2-hydroxy-1-(phenylmethyl)propyl]-N-4-methoxyphenylmethyl-2-methyl, [1S-[1R*(2R*),2S*]]-

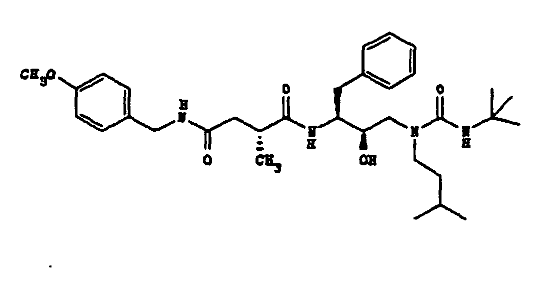

A 50 mL round bottomed flask was charged with 2(R)-methyl 4-N(4-methoxybenzyl)succinamide (588 mg, 2.35 mmol), N-hydroxybenzotriazole (511 mg, 3.34 mmol) and 6 mL of DMF. The solution was cooled to 0° C and treated with 1-(3-dimethylaminopropyl)-3-ethylcarbodiimide hydrochloride (502 mg, 2.62 mmol) for 20 m. A solution of (2R,3S)-3-amino-1-(3-methylbutyl)-1-[(1,1-dimethylethyl)amino]carbonyl)-4-phenyl-2-butanol (782 mg, 2.24 mmol) in 2 mL of DMF was added and the solution stirred at room temperature for a period of 24 h. The solution was concentrated *in vacuo* and poured into 50 mL of 50% sat. aq. NaHCO₃, the aqueous phase was extracted with CH₂Cl₂. The organic phase was washed with 5% citric acid, NaHCO₃, brine, dried over anhyd. MgSO₄, filtered and concentrated to give an oil that was purified by radial chromatography on SiO₂ eluting with hexane/ethyl acetate to give 790 mg, 59% of pure product as a white foam.

### Butanediamide, N'-[3-[[[(1,1-dimethylethyl)amino]carbonyl](3-methylbutyl)amino]-2-hydroxy-1-(phenylmethyl)propyl]-N-phenylmethyl-2-methyl, [1S-[1R*(2R*),2S*]]-

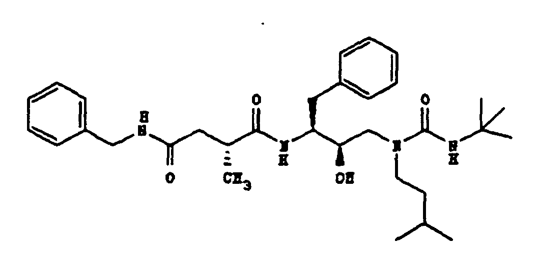

A 50 mL round bottomed flask was charged with 2(R)-methyl 4-N-(benzyl) succinamide (243 mg, 1.1 mmol), N-hydroxybenzotriazole (213 mg, 1.39 mmol) and 3 mL of DMF. The solution was cooled to 0° C and treated with 1-(3-dimethylaminopropyl)-3-ethylcarbodiimide hydrochloride (228 mg, 1.17 mmol) for 20 m. A solution of (2R,3S)-3-amino-1-(3-methylbutyl)-1-[(1,1-dimethylethyl)amino]carbonyl)-4-phenyl-2-butanol (327 mg, 0.95 mmol) in 2 mL of DMF was added and the solution stirred at room temperature for a period of 24 h. The solution was concentrated in vacuo and poured into 50 mL of 50% sat. aq. NaHCO₃, the aqueous phase was extracted with CH₂Cl₂. The organic phase was washed with 5% citric acid, NaHCO₃, brine, dried over anhyd. MgSO₄, filtered and concentrated to give an oil that was purified by flash chromatography on SiO₂ eluting with hexane/ethyl acetate to give 370 mg, 70% of pure product as a white foam.

### Example 2

Following the procedure generally as set forth in Example 1, as well as in Examples 4-17 the compounds shown in Table 14 were prepared.

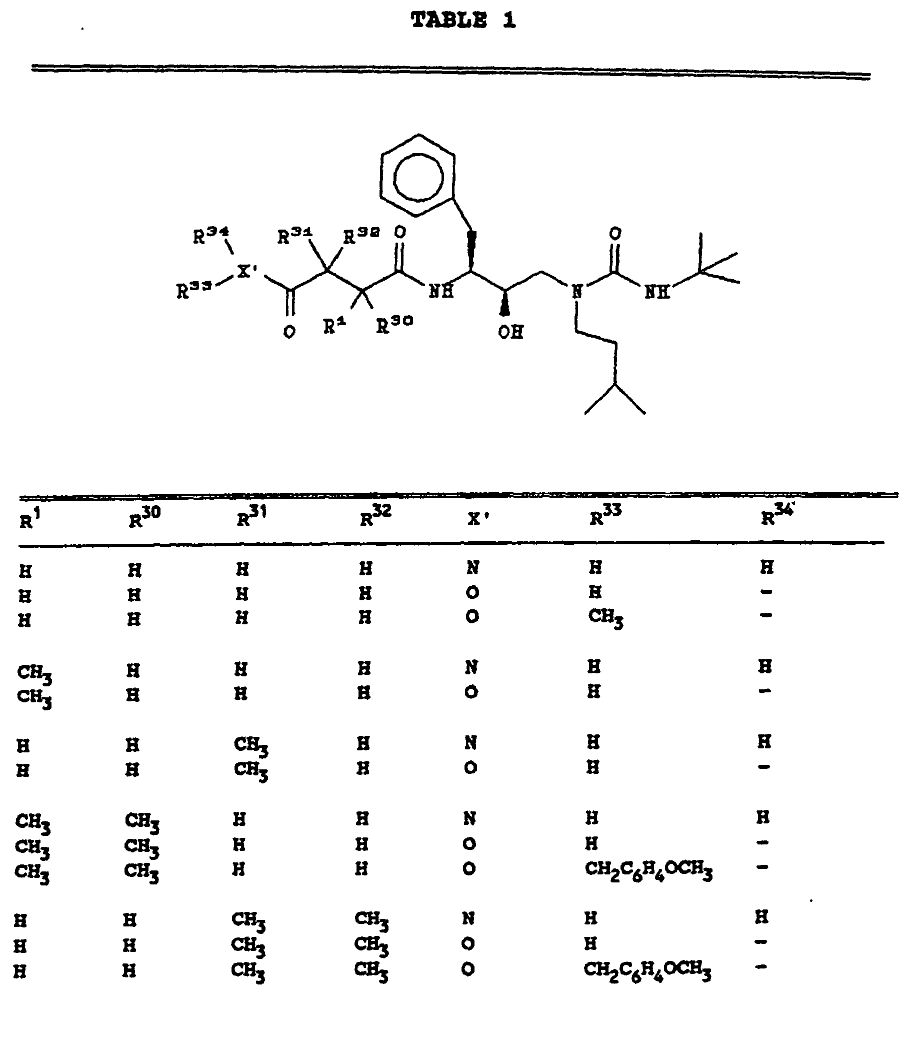

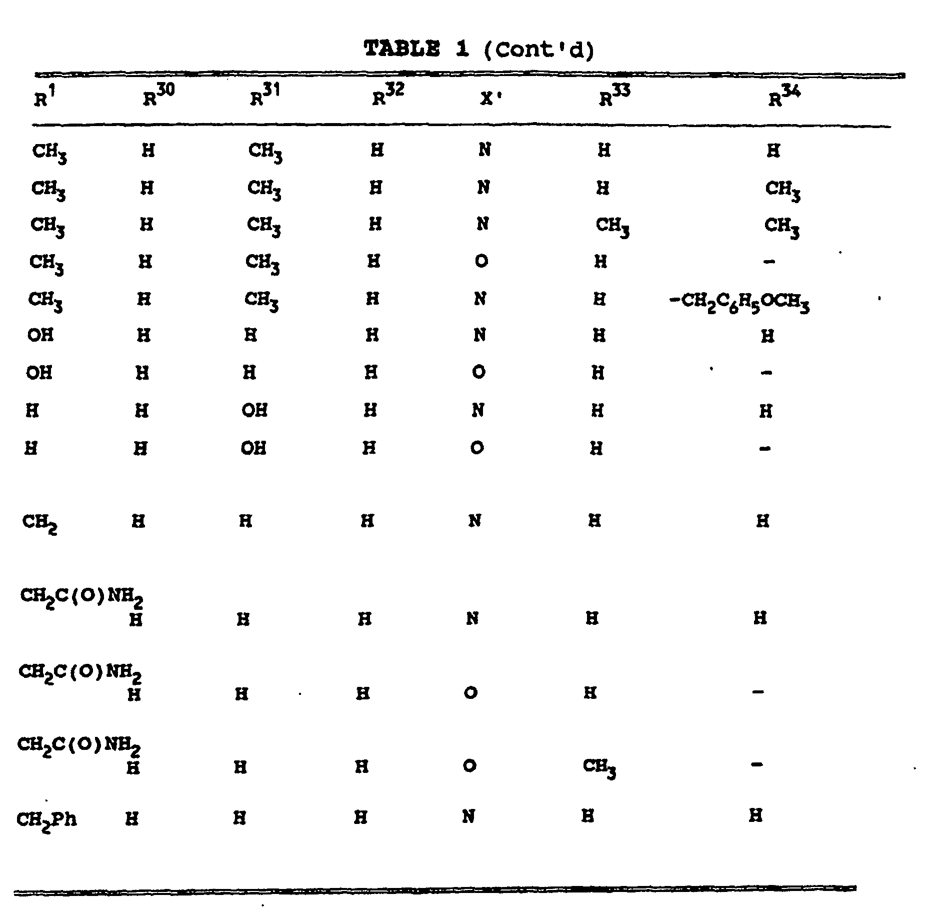

### Example 3

Following the procedure generally as set forth in Example 1, as well as Examples 4-17, the compounds shown in Table 2 were prepared.

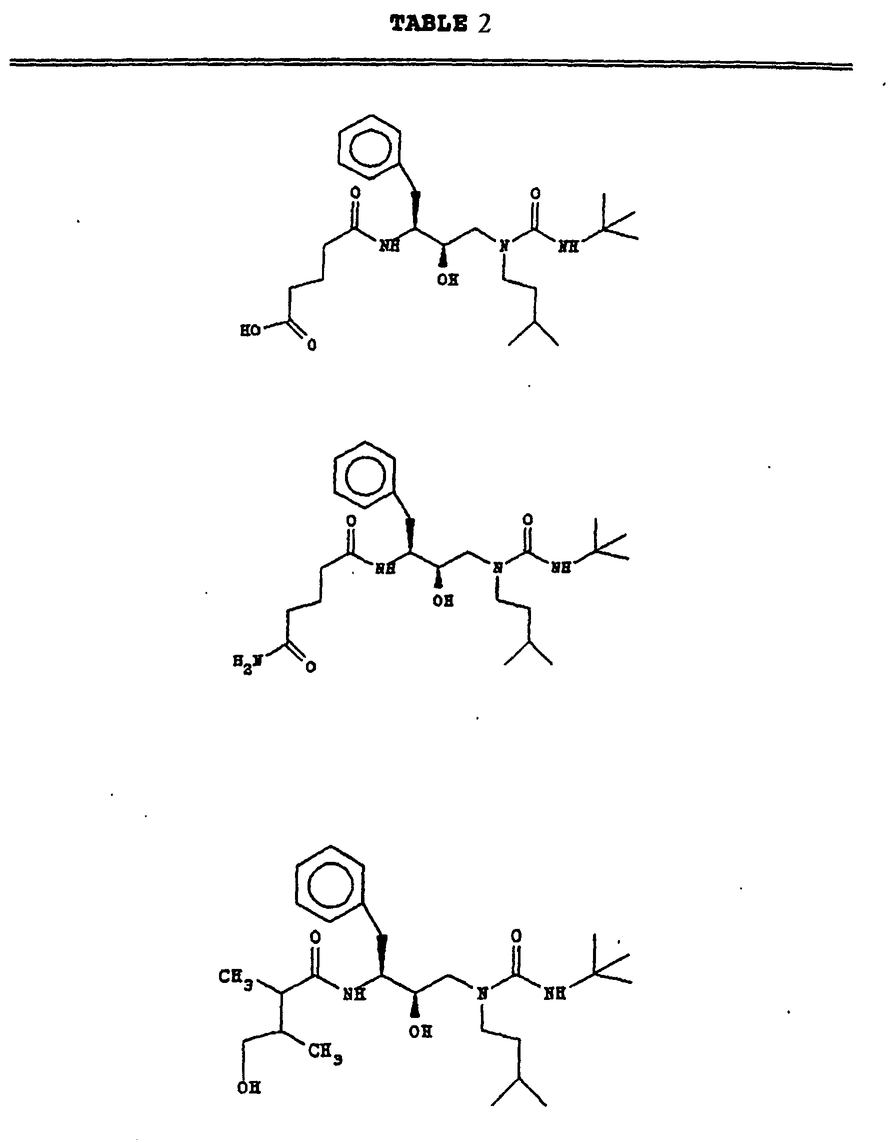

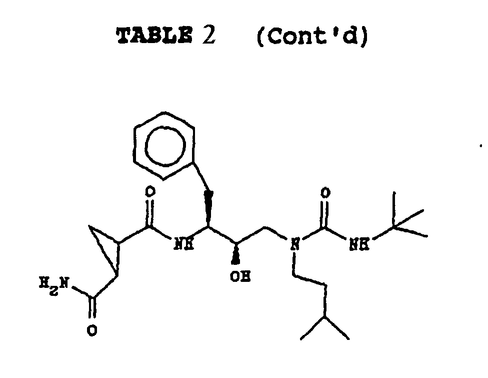

### Example 4

### Preparation of 3(S)-[N-(2-quinolinylcarbonyl)-L-asparaginyl]amino-2(R)-hydroxy-4-phenylbutylamine, N-(3-methylbutyl).

### Part A:

Preparation of N-3(S)-(Benzyloxycarbonyl)-amino-2(R)-hydroxy-4-phenylbutylamine, N-(3-methylbutyl). A solution of 20g (67 mmol) of N-benzyloxycarbonyl-3(S)-amino-1,2-(S)-epoxy-4-phenylbutane in 140 mL of isopropyl alcohol was treated with 83g (952 mmol) of isoamylamine and refluxed for one hour. The solution was cooled, concentrated, hexane added and the resulting solid filtered to afford 22.4g of the desired product.

### Part B:

Preparation of N-3(S)-(Benzyloxycarbonyl)-amino-2(R)-hydroxy-4-phenylbutylamine, N-(3-methylbutyl)-N-(t-butyloxycarbonyl). To a solution of 22.4g (58.3 mmol) of product from Part A above, 6.48g (64.1 mmol) of triethylamine and 150 mg of N,N-dimethyl-4-aminopyridine in 200 mL of tetrahydrofuran at 0°C was added 12.7g (58.3 mmol) of di-t-butylpyrocarbonate in 10 mL of THF. After 3.5 hours at room temperature, the volatiles were removed, ethyl acetate added and washed with 5% citric acid, sat d NaHCO₃, dried and concentrated to afford 30g of crude product. Chromatography on silica gel using 20% ethyl acetate/hexane afforded 22.5g (79%) of the desired product.

### Part C:

Preparation of N-3(S)-[N-benzyloxycarbonyl-L-asparaginyl]amino-2(R)-hydroxy-4-phenylbutylamine, N-(3-methylbutyl)-N-(t-butyloxycarbonyl). A solution of 22.5g of product from Part B above in 200 mL of ethanol was hydrogenated over 5.9g of 10% palladium-on-carbon under 50 psig hydrogen for one hour. The catalyst was filtered and the solvent removed under reduced pressureto afford 15.7g of free amine. This was dissolved in 130 mL of DMF and 4.54g (44.9 mmol) of N-methylmorpholine an added to a mixture of 13.3g (49.9 mmol) N-benzyloxy-carbonyl-L-asparagine, 11.5g (74.9 mmol) of N-hydroxybenzotriazole and 10.5g (54.9 mmol) of EDCl in 120 mL of DMF at 0°C, which had been preactivated for one hour prior to the addition. The mixture was stirred for 2 hours at 0°C and then for 12 hours at room temperature. The reaction was poured into 1L of sat d aqueous sodium bicarbonate, the solid collected, dissolved in ethyl acetate, washed with water, sat d sodium bicarbonate, 5% citric acid and brine, dried and concentrated to afford 16.7g of the desired product.

### Part D:

Preparation of N-3(S)-[N-(2-quinolinylcarbonyl)-L-asparaginyl]amino-2(R)-hydroxy-4-phenylbutylamine, N-(3-methylbutyl)-N-(t-butyloxycarbonyl). A solution of 16.7g (28.0 mmol) of product from Part C in 250 mL of methanol was hydrogenated over 6.0g of 10% palladium-on-carbon and under 50 psig hydrogen for one hour. The catalyst was filtered and the solution concentrated to afford 10.0g of free amine. This was dissolved in 100 mL of methylene chloride, 4.35g (43 mmol) of N-methylmorpholine was added followed by 5.53g (20.5 mmol) of quinoline-2-carboxylic acid, N-hydroxysuccinimide ester. This was stirred at room temperature overnight, the solvent removed, ethyl acetate added and washed with 5% citric acid, sat d sodium bicarbonate, brine, dried and concentrated to afford 14g of crude product. Recrystallization from ethyl acetate and hexane afforded 10.5g (83%) of desired product.

### Part E:

Preparation of N-3(S)-[N-(2-quinolinylcarbonyl)-L-asparaginyl]amino-2(R)-hydroxy-4-phenylbutylamine, N-(3-methylbutyl). To 80 mL of 4N hydrochloric acid in dioxane was added 9.17g (14.8 mmol) of product from Part D above. After one hour, the product becomes gummy. The solvents were removed, diethyl ether added and removed and the residue dissolved in 20 mL of methanol. This solution was added to 400 mL of sat d aqueous sodium bicarbonate, the solids collected, washed with acetone and hexane and dried in vacuo over P₂O₅ to afford 4.75g of the desired product.

### Example 5A

### Preparatioh of Benzyl 2,2,3(R)-trimethylsuccinate

### Part A:

Preparation of Methyl (S)-lactate, 2-methoxy-2-propyl ether. To a mixture of methyll(s)-(-)-lactate (13.2g, 100 mmol) and, 2-methoxypropene (21.6g, 300 mmol) in CH₂Cl₂ (150 ml) was added POCl₃ (7 drops) at r.t. and the resulting mixture was stirred at this temperature for 16 hours. After the addition of Et₃N (10 drops), the solvents were removed in vacuo to give 20.0g of (98%) desired product.

### Part B:

Preparation of 2(S)-hydroxypropanal, 2-methoxy-2-propyl ether. To a solution of compound from Part A (20.0g) in CH₂Cl₂ (100 ml) was added DIBAL (65 ml of 1.5M solution in toluene, 97.5 mmol) dropwise at -78°C for 45 min., then stirring was continued at the temperature for another 45 min. To this cold solution was added MeOH (20 ml), saturated NaCl solution (10 ml) and allowed the reaction mixture to warm up to r.t. and diluted with ether (200 ml), MgSO₄ (150g) was added and stirred for another 2 h. The mixture was filtered and the solid was washed twice with ether. The combined filtrates were rotavaped to afford 11.2g (78%) of the desired aldehyde.

### Part C:

Preparation of 2(S)-hydroxy-cis-3-butene, 2-methoxy-2-propyl ether. To a suspension of ethyltriphenylphosphonium bromide (28g, 75.5 mmol) in THF (125 ml) was added KN (TMS)₂ (15.7g, 95%, 75 mmol) in portions at 0°C and stirred for 1 h at the temperature. This red reaction mixture was cooled to -78°C and to this was added a solution of aldehyde from Part B (11g, 75 mmol) in THF (25 ml). After the addition was completed, the resulting reaction mixture was allowed to warm up to r.t. and stirred for 16 h. To this mixture was added saturated NH₄Cl (7.5 ml) and filtered through a pad of celite with a thin layer of silica gel on the top. The solid was washed twice with ether. The combined filtrates were concentrated in vacuo to afford 11.5g of crude product. The purification of crude product by flash chromatography (silica gel, 10:1 Hexanes/EtoAc) affording 8.2g (69%) pure alkene.

### Part D:

Preparation of 2(S)-hydroxy-cis-3-butene. A mixture of alkene from Part C (8.2g) and 30% aqueous acetic acid (25 ml) was stirred at r.t. for 1 hour. To this mixture was added NaHCO₃ slowly to the pH - 7, then extracted with ether (10 ml x 5). The combined ether solutions were dried (Na₂SO₄) and filtered. The filtrate was distilled to remove the ether to give 2.85g (64%) pure alcohol, m/e=87(M+H).

### Part E:

Preparation of 2,2,3-trimethyl-hex-(trans)-4-enoic acid. To a mixture of alcohol from Part D (2.5g, 29 mmol) and pyridine (2.5 ml) in CH₂Cl₂ (60 ml) was added isobutyryl chloride (3.1g, 29 mmol) slowly at 0°C. The resulting mixture was stirred at r.t. for 2 hours then washed with H₂O (30 ml x 2) and sat. NaCl (25 ml). The combined organic phases were dried (Na₂SO₄), concentrated to afford 4.2g (93%) ester 2(S)-hydroxy-cis-3-butenyl isobutyrate. This ester was dissolved in THF (10 ml) and was added to a 1.0M LDA soln. (13.5 ml of 2.0M LDA solution in THF and 13.5 ml of THF) slowly at -78°C. The resulting mixture was allowed to warm up to r.t. and stirred for 2 h and diluted with 5% NaOH (40 ml). The organic phase was separated, the aqueous phase was washed with Et₂O (10 ml). The aqueous solution was collected and acidified with 6N HCl to pH - 3. The mixture was extracted with ether (30 ml x 3). The combined ether layers were washed with sat. NaCl (25 ml), dried (Na₂SO4) and concentrated to afford 2.5g (60%) of desired acid, m/e=157(M+H).

### Part F:

Preparation of benzyl 2,2,3(S )-trimethyl-trans-4-hexenoate.A mixture of acid from Part E (2.5g, 16 mmol), BnBr (2.7g, 15.8 mmol), K₂CO₃ (2.2g, 16 mmol), NaI (2.4g) in acetone (20 ml) was heated at 75°C (oil bath) for 16 h. The acetone was stripped off and the residue was dissolved in H₂O (25 ml) and ether (35 ml). The ether layer was separated, dried (Na₂SO₄) and concentrated to afford 3.7g (95%) of benzyl ester, m/e=247(M+H).

### Part G:

Preparation of benzyl 2,2,3(R)-trimethylsuccinate. To a well-stirred mixture of KMnO₄ (5.4g, 34, 2 mmol), H₂O (34 ml), CH₂Cl₂ (6 ml) and benzyltriethylammonium chloride (200 mg) was added a solution of ester from Part F (2.1g, 8.54 mmol) and acetic acid (6 ml) in CH₂Cl₂ (28 ml) slowly at 0°C. The resulting mixture was stirred at the temperature for 2 h then r.t. for 16 h. The mixture was cooled in an ice-water bath, to this was added 6N HCl (3 ml) and solid NaHSO₃ in portions until the red color disappeared. The clear solution was extracted with CH₂Cl₂ (30 ml x 3). The combined extracts were washed with sat. NaCl solution, dried (Na₂SO₄) and concentrated to give an oil. This oil was dissolved in Et₂O (50 ml) and to this was added sat. NaHCO₃ (50 ml). The aqueous layer was separated and acidified with 6N HCl to pH - 3 then extracted with Et₂O (30 ml x 3). The combined extracts were washed with sat. NaCl solution (15 ml), dried (Na₂SO₄) and concentrated to afford 725 mg (34%) of desired acid, benzyl 2,2,3(R)-trimethylsuccinate, m/e=251(M+H).

### Example 5B

### Part A:

### Preparation of Butanediamide, N¹-[3-[[[(1,1-dimethylethyl)amino]carbonyl](3-methylbutyl)amino]-2-hydroxy-1-(phenylmethyl)propyl]-2,3,3-trimethyl-[1S-, [1R*(2S*),2S*]]-

To a well-stirred solution of benzyl 2,2,3(R)-trimethylsuccinate (225 mg, 0.9 mmol) in DMF (1.0 ml) was added HOBt (230 mg, 1.5 mmol). The clear reaction mixture was then cooled to 0°C, to this was added EDC (210 mg, 1.1 mmol) and stirred for 1 h at the temperature. To this cold mixture was added a powder of (350 mg, 1.0 mmol) and DMF (0.5 ml). The resulting reaction mixture was stirred for 2 h at 0°C and 16 h at r.t. After the removal of DMF (≤ 40°C), a solution of 60% sat. NaHCO₃ (10 ml) was added. This mixture was extracted with EtOAc (10 ml x 2). The extracts were combined and washed with sat. NaHCO₃ (10 ml x 2), 5% citric acid (10 ml x 2), H₂O (10 ml), sat. NaCl (10 ml) and dried (Na₂SO₄) then concentrated to afford 512 mg (98%) of desired product Butanoic Acid, 4-[[3-[[[(1,1-dimethylethyl)amino]carbonyl](3-methylbutyl)amio]-2-hydroxy-1-(phenylmethyl)propyl]amino]-2,2,3-trimethyl-4-oxo, [1S-[1R*(3S*),2S*]]-benzyl ester as a white solid, m/e=582(M+H).

### Part B:

A mixture of benzyl ester from Part A (480 mg, 0.825 mmol), 10% Pd/C (450 mg) in MeOH (25 ml) was hydrogenated (H₂, 50 psi) for 1/2 h at r.t. The mixture was filtered and the solid was washed with MeOH (10 ml). The collected filtrates were concentrated to afford a crude acid as a white solid. The crude acid was dissolved in Et₂O-EtOAc (10:1, 25 ml) and the solution was washed with sat. NaHCO₃ (25 ml) then 5% NaOH (10 ml). The combined aqueous layers were cooled to 0°C and acidified with concentrated HCl (Co₂) to pH ∼ 1 then extracted with Et₂O-EtOAC (10:1, 25 ml x 3). The combined extracts were washed with sat. NaCl (15 ml), dried (Na₂SO₄) and concentrated to afford 307 mg (75.7%) of pure acid Butanoic acid, 4-[[3-[[[(1,1-dimethylethyl)amino]carbonyl](3-methylbutyl)amino]-2-hydroxy-1-(phenylmethyl)propyl]amino]-2,2,3-trimethyl-4-oxo-,[1S-[1R*(3S*),2S*]]-, as a white solid, m/e=491(M+H).

### Part C:

### Butanoic acid, 4-[[3-[[[(1,1-dimethylethyl)amino]carbonyl](3-methylbutyl)amino]-2-hydroxy-1-(phenylmethyl)propyl]amino]-2,2,3-trimethyl-4-oxo-,[1S-[1R*(3S*),2S*]]-, as a white solid, m/e=491(M+H).

To a well-stirred solution of the acid from Part B (245 mg, 0.5 mmol) in DMF (0.5 ml) was added HOBt (153 mg, 1.0 mmol) and EDC (143 mg, 0.75 mmol) at 0°C. After stirring at 0°C for 2 h, NH₄OH (0.63 ml of 28% NH₄OH, 5 mmol) was added and stirred at 0°C for 2 h, r.t. for 16 h. The removal of DMF (≤ 40°C) gave a white solid. The purification of the crude product by flash chromatography (silica gel, 5% MeOH/CH₂Cl₂) gave 172 mg (70%) of pure amide as a white solid, m/e=491(M+H).

### Example 6

### Preparation of methyl 2,2-dimethyl-3-methyl succinate, (R) and (S) isomers.

### Part A:

Preparation of methyl 2,2-dimethyl-3-oxo-butanoate. A 250 ml RB flask equipped with magnetic stir bar and N₂ inlet was charged with 100 ml dry THF and 4.57g (180 mmol) of 95% NaH. The slurry was cooled to -20°C and 10g (87 mmol) methyl acetoacetate was added dropwise followed by 11.3 ml (181 mmol) CH₃I. The reaction was stirred at 0°C for 2 hours and let cool to room temperature overnight. The reaction was filtered to remove NaI and diluted with 125 ml Et₂O. The organic phase was washed with 1x100 1 5% brine, dried and concentrated in vacuo to a dark golden oil that was filtered through a 30g plug of silica gel with hexane. Concentration in vacuo yielded 10.05g of desired methyl ester, as a pale yellow oil, suitable for use without further purification.

### Part B:

Preparation of methyl 2,2-dimethyl-3-0-(trifluoromethanesulfonate)-but-3-enoate. A 250 ml RB flask equipped with magnetic stir bar and N₂ inlet was charged with 80 mL by THF and 5.25 ml (37.5 mmol) diisopropylamine was added. The solution was cooled to -25°C (dry ice/ethylene glycol) and 15 ml (37.5 mmol) of 2.5 M n-BuLi in hexanes was added. After 10 minutes a solution of 5g (35 mmol) 1 in 8 ml dry THF was added. The deep yellow solution was stirred at -20°C for 10 min. then 12.4g N-phenyl bis(trifluoromethanesulfonimide) (35 mmol) was added. The reaction was stirred @ -10°C for 2 hours, concentrated in vacuo and partioned between ethyl acetate and sat. NaHCO₃. The combined organic phase was washed with NaHCO₃, brine and conc. to an amber oil that was filtered through 60g silica gel plug with 300 mL 5% ethyl acetate/hexane Conc. in vacuo yielded 9.0g light yellow oil that was diluted with 65 ml ethyl acetate and washed with 2x50 ml 5% aq K₂CO₃, 1x10 mL brine, dried over Na₂SO₄ and conc. in vacuo to yield 7.5g (87%) vinyl triflate, (m/e=277(M+H) suitable for use without further purification.

### Part C:

Preparation of methyl 2,2-dimethyl-3-carboxyl-but-3-enoate. A 250 ml Fisher Porter bottle was charged with 7.5g (27 mmol) of compound prepared in B, 50 ml dry DMF, 360 mg (1.37 mmol) triphenyl phosphine and 155 mg (.69 mmol) Pd(II)(OAc)₂. The reaction mixture was purged twice with N₂ then charged with 30 psi CO. Meanwhile a solution of 20 ml dry DMF and 7.56 ml (54 mmol) NEt₃ was cooled to 0°C to this was added 2.0g (43 mmol) of 99% formic acid. The mixture was swirled and added to the vented Fisher Porter tube. The reaction vessel was recharged to 40 psi of CO and stirred 6 hours @ room temperature. The reaction mixture was concentrated in vacuo and partionned between 100 mL of ethyl acetate and 75 mL 5% aq K₂CO₃. The aqueous phase was washed with 1x40 mL additional ethyl acetate and then acidified with conc. HCl/ice. The aqueous phase was extracted with 2x70 mL of ethyl acetate and the organics were dried and conc. to yield 3.5g (75%) white crystals, mp 72-75°C, identified as the desired product (m/e=173(M+H).

### Part D:

Preparation of methyl 2,2-dimethyl-3-methylsuccinate, isomer #1. A steel hydrogenation vessel was charged with 510 mg (3.0 mmol) acrylic acid, from Part C, and 6 mg Ru (acac)₂ (R-BINAP) in 10 ml degassed MeOH. The reaction was hydrogenated at 50 psi/room temperature for 12 hours. The reaction was then filtered through celite and conc. to 500 mg clear oil which was shown to be a 93:7 mixture of isomer #1 and #2, respectively as determined by GC analysis using a 50 M β-cyclodextrin column: 150°C - 15 min. then ramp 2°C/min.; isomer #1, 17.85 min., isomer #2, 18-20 min.

### Part E:

Preparation of methyl 2,2-dimethyl-3-methylsuccinate, Isomer #2. A steel hydrogenation vessel was charged with 500 mg (2.9 mmol) acrylic acid, Part C, and 6 mg Ru(OAc) (acac)(S-BINAP) in 10 ml degassed MeOH. The reaction was hydrogenated at 50 psi/room temperature for 10 hours. The reaction was filtered through celite and concentrated in vacuo to yield 490 mg of product as a 1:99 mixture of isomers #1 and #2, respectively, as determined by chiral GC as above.

### Example 7

### Preparation of 3-[[[(1,1-dimethylethyl)amino]carbonyl](3-methylbutyl)amino]-2(R)-hydroxy-1(S)-(phenylmethyl)propylamine, 1.

### Part A:

To a solution of 75.0g (0.226 mol) of N-benzyloxycarbonyl-L-phenylalanine chloromethyl ketone in a mixture of 807 mL of methanol and 807 mL of tetrahydrofuran at -2°C, was added 13.17g (0.348 mol, 1.54 equiv.) of solid sodium borohydride over one hundred minutes. The solvents were removed under reduced pressure at 40°C and the residue dissolved in ethyl acetate (approx. 1L). The solution was washed sequentially with 1M potassium hydrogen sulfate, saturated sodium bicarbonate and then saturated sodium chloride solution. After drying over anhydrous magnesium sulfate and filtering, the solution was removed under reduced pressure. To the resulting oil was added hexane (approx. 1L) and the mixture warmed to 60°C with swirling. After cooling to room temperature, the solids were collected and washed with 2L of hexane. The resulting solid was recrystallized from hot ethyl acetate and hexane to afford 32.3g (43% yield) of N-benzyloxycarbonyl-3(S)-amino-1-chloro-4-phenyl-2(S)-butanol, mp 150-151°C and M+Li⁺ = 340.

### Part B:

To a solution of 6.52g (0.116 mol, 1.2 equiv.) of potassium hydroxide in 968 mL of absolute ethanol at room temperature, was added 32.3g (0.097 mol) of N-CBZ-3(S)-amino-1-chloro-4-phenyl-2(S)-butanol. After stirring for fifteen minutes the solvent was removed under reduced pressure and the solids dissolved in methylene chloride. After washing with water, drying over magnesium sulfate, filtering and stripping, one obtains 27.9g of a white solid. Recrystallization from hot ethyl acetate and hexane afforded 22.3g (77% yield) of N-benzyloxycarbonyl-3(S)-amino-1,2(S)-epoxy-4-phenylbutane, mp 102-103°C and MH⁺ 298.

### Part C:

A solution of N-benzyloxycarbonyl 3(S)-amino-1,2-(S)-epoxy-4-phenylbutane (30.1g, 0.10 mol) and 165mL of isoamylamine in 150 mL of isopropyl alcohol was heated to reflux for 2.5 hours. The solution was cooled to room temperature, concentrated in vacuo and then recrystallized. The product was isolated by filtration and from ethyl acetate/hexane to afford 31.7g (81%) of N[3(S)-benzyloxycarbonylamino-2(R)-hydroxy-4-phenylbutyl]N-isoamylamine.

### Part D:

A solution of N[3(S)-benzyloxycarbonylamino-2(R)-hydroxy-4-phenyl butyl], N-isoamylamine in 10 ml of tetrahydrofuran was treated with tert-butylisocyanate (267 mg, 2.70 mmol) at room temperature for 5 minutes. The solvent was removed in vacuo and replaced with ethyl acetate. The ethyl acetate solution was washed with 5% citric acid, water, and brine, dried over anhydrous MgSO₄, filtered and concentrated in *vacuo* to give 1.19g, 97% of N-benzyloxycarbonyl-3-[[[(1,1-dimethylethyl)amino]carbonyl](3-methylbutyl)amino]-2(R)-hydroxy-1(S)-(phenylmethyl)propylamine, MH⁺ m/z = 470.

### Part E:

A solution of (37.3g, 77 mmol) of product from Part D in 100 mL of methanol was hydrogenated over 10% palladium-on-carbon for 4 hours to afford 26.1g of the desired product.

### Example 8

### Preparation of Butanediamide, N-[3-[[[(1,1-dimethylethyl)amino]carbonyl](3-methylbutyl)amino]-2-hydroxy-1-(phenylmethyl)propyl]-, [1S-[1R*, 2S*]]-.

### Part A:

To a solution of 102mg (0.29 mmol) of Example 7 Part E and 70 mg (0.89 mmol) of pyridine in 2 mL of methylene chloride was added 29 mg (0.29 mmol) of succinic anhydride. After 2 hours, ethyl acetate was added and then extracted with saturated NaHCO. The aqueous layer was acidified, reextracted with ethyl acetate, washed with saturated brine, dried over magnesium sulfate, filtered and concentrated *in vacuo* to afford 78 mg (60%) of butanoic acid, 4-[[3-[[[(1,1-dimethylethyl)amino]carbonyl](3-methylbutyl)amino]-2-hydroxy-1-(phenylmethyl)propyl]amino-4-oxo-, [1S-[1R*, 2S*]-.

### Part B:

This was activated with EDC and N-hydroxybenzotriazole in N,N-dimethylformamide and then reacted with ammonia to generate the desired final compound.

### Example 9

### Part A:

To a solution of 4.60g (24.7 mmol) of trans-diethyl 1,2-cyclopropanedicarboxylate in 100 mL of 50:50 v:v tetrahydrofuran/water was added 1.24g (29.6 mmol) of lithium hydroxide. After 17 hours, the tetrahydrofuran was removed *in vacuo,* the water layer washed with ethyl acetate, acidified with 1N hydrochloric acid and reextracted with ethyl acetate. The organic layer was dried and stripped to afford 2.1g of crude product. After recrystallization from diethyl ether/hexane and then methylene chloride/hexane one obtains 1.1g (28%) of trans-monoethyl 1,2-cyclopropanedicarboxylate, m/e = 159 (M + H).

### Part B:

To a solution of 297 mg (1.87 mmol) of trans-monoethyl 1,2-cyclopropanedicarboxylate and 429 mg (2.8 mmol) N-hydroxybenzotriazole (HOBT) in 3 mL of anhydrous N,N-dimethylformamide (DMF) at 0°C was added 394 mg (2.0 mmol) of 1-(3-dimethylaminopropyl)-3-ethylcarbodiimide hydrochloride (EDC). After 30 min. a solution of 591 mg (1.7 mmol) of product from Part A in 2 mL DMF and 171 mg (1.69 mmol) of N-methylmorpholine (NMM) was added. After 2 hours at 0°C, the reaction was stirred at RT overnight, poured into water, extracted with ethyl acetate, washed with water, 5% aq. citric acid, sat'd NaHCO₃, brine, dried over anhydride MgSO₄ and stripped to afford 771 mg of crude product. This was chromatographed on silica gel using 5-20% methanol/methylene chloride to afford 670 mg (80%) of cyclopropane carboxylic acid, 2-[[[3-[[[(1,1-dimethylethyl)amino]carbonyl](3-methylbutyl)amino]-2-hydroxy-1-(phenylmethyl)propyl]amino]carbonyl]-, ethyl ester; m/e = 490 (M + H).

### Part C:

To a solution bf 658 mg (1.32 mmol) of product from Part B in 5 mL of 50:50 THF/water was added 66 mg (1.58 mmol) of lithium hydroxide. After 19 hours, the THF was removed in vacuo, the water washed with ethyl acetate, acidified and reextracted with ethyl acetate. The organic layer was dried and stripped to afford 328 mg (54%) of the corresponding acid, m/e = 462 (M + H).

### Part D:

To a solution of 304 mg (0.66 mmol) of product from Part C, 151 mg (0.99 mmol) HOBT in 2.2 mL DMF at 0°C was added 139 mg (0.73 mmol) EDC. After 30 min. at 0°C, 1.1 mL of conc. aqueous ammonia was added. After stirring at 0°C for 2 hours and RT for 20 hours, the reaction was poured into brine and extracted with ethyl acetate. After washing with sat'd NaHCO₃, brine, drying and stripping, one obtains 141 mg of crude product. This was chromatographed on silica gel with 1-5% methanol/methylene chloride to afford 40 mg (13%) of the desired final product, m/e = 561 (M + H).

### Example 10

### Preparation of trans-but-2-enediamide, N-[3-[[[(1,1-dimethylethyl)amino]carbonyl](3-methylbutyl)amino]-2-hydroxy-1-(phenylmethyl)propyl]-, [1S-[1R*, 2S*].

### Part A:

To a solution of 137 mg (0.95 mmol) fumaric acid monoethyl ester in 1 mL of DMF at 0°C was added 183 mg (0.95 mmol) EDCl. After 15 minutes, a solution of 333 mg (0.95 mmol) of the compound of Example 34 Part E in 1 mL DMF was added and the reaction stirred for 14 hours at RT. Ethyl acetate was added and extracted with sat'd brine, 0.2 n HCl, sat'd NaHCO₃, dried and stripped to afford 0.32g of crude product. Chromatography on silica gel using 0-50% ethyl acetate/hexane afforded 0.26g (58%) of but-2-enoic acid, 4-[[3-[[[(1,1-dimethylethyl)amino]carbonyl](3-methylbutyl)amino]-2-hydroxy-1-(phenylmethyl)propyl]amino]-4-oxo-, [1S-[1R*, 2S*]]-, ethyl ester, m/e = 476 (M + H).

### Part B:

To a solution of 26.6 mg (0.56 mmol) of product from Part A in 3 mL of 50:50 THF/water was added 34 mg (0.82 mmol) of lithium hydroxide and the reaction stirred at RT for 1 hour. The THF was removed in vacuo, the aqueous layer acidified with 1N HCl and extracted with ethyl acetate. The organic layer was washed with brine, dried and stripped to afford 233 mg (93%) of trans-but-2-enoic acid, 4-[[3-[[[(1,1-dimethylethyl)amino]carbonyl](3-methylbutyl)amino]-2-hydroxy-1-(phenylmethyl)propyl]amino]-4-oxo-, [1S-[1R*, 2S*]-, m/e = 448 (M + H).

### Part C:

To a solution of 225 mg (0.50 mmol) of the product from Part B in 1 mL of DMF was added 95 mg (0.50 mmol) EDC. After 15 minutes at RT, 0.50 mL of conc. aqueous ammonia was added and the reaction stirred for 15 hours. Ethyl acetate was added and washed with 0.2N HCl, brine, dried and stripped to afford 170 mg of crude product. After chromatography on silica gel using 0-40% methanol/methylene chloride, one obtains 50 mg (22%) of trans-but-3-enediamide, N-[3-[[[(1,1-dimethylethyl)amino]carbonyl](3-methylbutyl)amino]-2-hydroxy-1-(phenylmethyl)propyl]-, [1S-[1R*, 2S*]-, m/e = 447 (M + H).

### Example 11

### Preparation of butanediamide, N-[3-[[[(1,1-dimethylethyl)amino]carbonyl](3-methylbutyl)amino]-2-hydroxy-1-(phenylmethyl)propyl-2-methyl-, [1S-[1R*(2S*), 2S*]-.

### Part A:

To a suspension of 24.7g (0.22 mol) of itaconic anhydride in 100 mL of anhydrous toluene at reflux under a nitrogen atmosphere was added dropwise over 30 minutes 23.9g (0.22 mol) of benzyl alcohol. The insoluble material dissolved to provide a homogeneous solution which was refluxed for 1.5 hours. The solution was cooled to RT, then in an ice bath and the resulting white precipitate collected by filtration to afford 24.8g (51%) of 4-benzyl itaconate.

### Part B:

To a solution of 2.13g (9.5 mmol) of the product from Part A in 12 mL of methylene chloride at 0°C was added 4.02g (29.1 mmol) of para-methoxybenzyl alcohol, 605 mg (4.95 mmol) of N,N-dimethyl 4-aminopyridine, 128 mg of N,N-dimethyl 4-aminopyridine hydrochloride salt and then 2.02g (4.7 mmol) dicyclohexylcarbodiimide (DCC). After stirring at 0°C for 1 hour and then RT for 2 hours, the precipitate was collected and discarded. The filtrate was washed with 0.5 N HCl, sat'd NaHCO₃, dried and stripped to afford 4.76g of crude product. This was chromatographed on silica gel using 0-50% ethyl acetate/hexane to afford 1.24g of pure 4'-methoxybenzyl-4-benzylitaconate , MH⁺ m/z = .

### Part C:

A solution of 1.24g (3.65 mmol) of product from Part B and 20 mg of [(R,R)-Dipamp)cyclooctadienylrhodium] tetrafluoroborate in 30 mL of methanol was throughly degassed, flushed with nitrogen and then hydrogen and then stirred under 50 psig of hydrogen for 15 hours. The solution was filtered and stripped, dissolved in methylene chloride and washed with sat'd NaHCO₃, dried and stripped to afford 0.99g of a brown oil. This was then dissolved in 40 mL of methylene chloride, 3 mL of trifluoroacetic acid added and the solution stirred at RT for 3.5 hours. Water was added and separated and the organic layer extracted with sat'd NaHCO₃. The aqueous layer was acidified and reextracted with ethyl acetate, separated and the organic layer washed with brine, dried and stripped to afford 320 mg (50%) of 2(R)-methyl-4-benzylsuccinic acid.

### Part D:

To a solution of 320 mg (1.44 mmol) of product from Part C and 314 mg (2.05 mmol) HOBT in DMF at 0°C was added 303 mg (1.58 mmol) of EDC. After stirring for 30 minutes, a solution of 467 mg (1.34 mmol) of the product from Example 34, Part E in 4 mL of DMF was added. After stirring for 1 hour at 0°C and 14 hours at RT, ethyl acetate was added and washed with sat'd NaHCO₃, 5% aqueous citric acid, dried and stripped to afford 0.97g of crude product. This was chromatographed on silica gel using 0-10% ethyl acetate/hexane to afford 420 mg of pure butanoic acid, 4-[[3-[[[(1,1-dimethylethyl)amino]carbonyl](3-methylbutyl)amino]-2-hydroxy-1-(phenylmethyl)propyl]amino]-3-methyl-4-oxo-, [1S-[1R*(3S*), 2S*]-, benzyl ester.

### Part E:

A solution of 150 mg (0.27 mmol) of product from Part D in 15 mL of methanol was hydrogenated over 10% palladium on carbon under 50 psig hydrogen for 17 hours. The reaction was filtered and stripped to afford 125 mg (100%) of butanoic acid, 4-[[3-[[[(1,1-dimethylethyl)amino]carbonyl](3-methylbutyl)amino]-2-hydroxy-1-(phenylmethyl)propyl]amino]-3-methyl-4-oxo-, [1S-[1R*(3S*), 2S*]-.

### Part F:

To a solution of 125 mg (0.27 mmol) of product from Part E and 65 mg (0.42 mmol) of HOBT in 5 mL of DMF at 0°C was added 59 mg (0.31 mmol) of EDC. After 30 min. at 0°C, 1 mL of conc. aqueous ammonia was added. After stirring at 0°C for 2 hours and RT fro 15 hours, ethyl acetate was added and washed with sat'd NaHCO₃, 5% aqueous citric acid, dried and stripped to afford 90 mg of crude product. This was recrystallized from ethyl acetate/hexane to afford 40 mg (32%) of pure butanediamide, N-[3-[[[(1,1-dimethylethyl)amino]carbonyl](3-methylbutyl)amino]-2-hydroxy-1-(phenylmethyl)propyl]-2-methyl, [1S-[1R*(2S*), 2S*]-.

### Example 12

### Preparation of butanediamide, N-[3-[[[(1,1-dimethylethyl)amino]carbonyl](3-methylbutyl)amino]-2-hydroxy-1-(phenylmethyl)propyl]-2-methyl, [1S-[1R*(2R*), 2S*]-.

### Part A:

A solution of 1.41g (4.1 mmol) of 4'-methoxybenzyl 4-benzylitaconate and 25 mg of [(S,S-Dipamp)cyclooctadienylrhodium]tetrafluoroborate in 20 mL of methanol was thoroughly degassed, flushed with nitrogen and then hydrogen and then stirred under 40 psig hydrogen for 72 hours. The solution was filtered and concentrated to provide 1.34g of a brown oil. This was dissolved in 40 mL of methylene chloride and 3 mL of trifluoroacetic acid was added. After stirring for 4 hours, water was added, separated and the organic layer extracted with sat'd NaHCO₃. The aqueous layer was separated, reacidified, extracted with ethyl acetate which was separated, washed with brine, dried and stripped to afford 440 mg of 2(S)-methyl-4-benzylsuccinic acid.

### Part B:

To a solution of 440 mg (1.98 mmol) of the product from Part A and 437 mg (2.86 mmol) of HoBT in 9 mL of DMF at 0°C was added 427 mg (2.23 mmol) of EDCl. After 30 minutes at 0°C, a solution of 653 mg (1.87 mmol) of the product from Example 34, Part E in 3 mL DMF was added. After 1 hour at 0°C and 15 hours at RT, ethyl acetate was added, extracted with sat'd NaHCO₃, 5% aqueous citric acid, dried and concentrated to afford 0.98g of crude product. Chromatography on silica gel using 0-10% ethyl acetate afforded 610 mg (59%) of pure butanoic acid, 4-[[3-[[[(1,1-dimethylethyl)-amino]carbonyl](3-methylbutyl)amino]-2-hydroxy-1-(phenylmethyl)propyl]amino]-3-methyl-4-oxo-, [1S-[1R*(3R*), 2S*], benzyl ester.

### Part C:

A solution of 310 mg (0.56 mmol) of the product from Part B in 20 mL of methanol was hydrogenated over 20 mg of 10% palladium on carbon under 50 psig hydrogen for 19 hours. The solution was filtered and concentrated to afford 220 mg (85%) of butanoic acid, 4-[[3-[[[(1,1-dimethylethyl)-amino]carbonyl](3-methylbutyl)amino]-2-hydroxy-1-(phenylmethyl)propyl]amino]-3-methyl-4-oxo-, [1S-[1R*(3R*), 2S*].

### Part D:

To a solution of 190 mg (0.41 mmol) of the product from Part C and 90 mg (0.58 mmol) HOBT in 5 mL of DMF at 0°C, was added 88 mg (0.46 mmol) of EDC.
After 30 minutes at 0°C, 2 mL of conc. aqueous ammonia was added. After 1 hour at 0°C and 15 hours at RT, ethyl acetate was added, washed with sat'd NaHCO₃, 5% aqueous citric acid, dried and concentrated to afford crude product. Recrystallization from ethyl acetate/hexane afforded 20 mg (11%) of butanediamide, N-[3-[[[(1,1-dimethylethyl)amino]carbonyl](3-methylbutyl)amino]-2-hydroxy-1-(phenylmethyl)propyl]-2-methyl, [1S-[1R*(2R*), 2S*]-.

### Example 13

### Preparation of butanediamide, N-[3-[[[(1,1-dimethylethyl)amino]carbonyl](3-methylbutyl)amino]-2-hydroxy-1-(phenylmethyl)propyl]-3-methyl-, [1S-[1R*(3S*), 2S*]-.

### Part A:

In a similar manner to the procedure used above, p-methoxybenzyl alcohol was reacted with itaconic anhydride in refluxing toluene to provide 4-(p-methoxybenzyl)itaconate.

### Part B:

To a solution of 3.30g (13.2 mmol) of the product from Part A in 17 mL of toluene, was added 2.08g (13.7 mmol) of 1,8-diazabicyclo[5.40]undec-7-enc and then 2.35g (13.7 mmol) of benzyl bromide. After 2 hours, the solution was filtered and the filtrate washed with sat'd NaHCO₃, 3N HCl, brine, dried and concentrated to afford 3.12g of an oil. After chromatography on silica gel using 0-5% ethyl acetate/hexane one obtains 2.19g (49%) of benzyl 4-(4-methoxybenzyl)itaconate.

### Part C:

A solution of 1.22g (3.6 mmol) of product from Part B and 150 mg of [((R,R-Dipamp)) cyclooctadienylrhodium] tetrafluoroborate in 15 mL of methanol was thoroughly degassed, flushed with nitrogen and then hydrogen and hydrogenated under 50 psig for 16 hours. The solution was filtered and concentrated to afford 1.2g of a brown oil. This was dissolved in 5 mL of methylene chloride and 5 mL of toluene and 3 mL of trifluoroacetic acid was added. After 4 hours, the solvents were removed *in vacuo*, the residue dissolved in methylene chloride, which was then extracted with sat'd NaHCO₃. After separation, the aqueous layer was acidified, reextracted with methylene chloride which was then dried and concentrated to afford 470 mg (60%) of 3(R)-methyl-4-benzylsuccinic acid.

### Part D:

To a solution of 470 mg (2.11 mmol) of product from Part C and 463 mg (3.03 mg) of HOBT in 5 mL of DMF at 0°C was added 451 mg (2.35 mmol) of EDC. After 30 min. at 0°C, a solution of 728 mg (2.08 mmol) of the product from Example 34, Part E in 3 mL of DMF was added. After stirring at 0°C for 1 hour and 15 hours at RT, ethyl acetate was added and extracted with sat'd NaHCO₃, 5% aqueous citric acid, brine, dried and concentrated to give 930 mg of crude product chromatography on silica gel using 0-10% ethyl acetate/hexane one obtains 570 mg (50%) of butanoic acid, 4-[[3-[[[(1,1-dimethylethyl)amino]carbonyl](3-methylbutyl)amino]-2-hydroxy-1-(phenylmethyl)propyl]amino]-2-methyl-4-oxo-, [1S-[1R*(2S*), 2S*]-, benzyl ester.

### Part E:

The product was hydrogenated in methanol using 10% palladium on carbon under 40 psig of hydrogen to afford butanoic acid, 4-[[3-[[[(1,1-dimethylethyl)amino]carbonyl]-(3-methylbutyl)amino]-2-hydroxy-1-(phenylmethyl)propyl]amino]-2-methyl-4-oxo-, [1S-[1R*(2S*), 2S*]-.

### Part F:

To a solution of 427 mg (0.92 mmol) of product from Part E and 210 mg (1.37 mmol) in 3 mL of DMF at 0°C was added 196 mg (1.02 mmol) of EDC. After 30 min. at 0°C, 2 mL of conc. aqueous ammonia was added. After 1 hour at 0°C and 15 hours at RT, ethyl acetate was added and then extracted with sat'd NaHCO₃, brine, dried and concentrated to afford crude product. Recrystallization from ethyl acetate/hexane afforded 50 mg (12%) of butanediamide, N-[3-[[[(1,1-dimethylethyl)amino]carbonyl](3-methylbutyl)amino]-2-hydroxy-1-(phenylmethyl)propyl]-3-methyl-, [1S-[1R*(3S*), 2S*]-.

### Example 14

### Preparation of butanediamide, N-[3-[[[(1,1-dimethylethyl)amino]carbonyl](3-methylbutyl)amino]-2-hydroxy-1-(phenylmethyl)propyl]-3-methyl-, [1S-[1R*(3R*), 2S*]-.

This was prepared in an identical manner to the previous example except that the asymmetric hydrogenation step was done in the presence of [((S,S-dipamp)cyclooctadienyl)rhodium]-tetrafluoroborate as catalyst.

### Example 15

### Preparation of butanediamide, N-[3-[[[(1,1-dimethylethyl)amino]carbonyl](3-methylbutyl)amino]-2-hydroxy-1-(phenylmethyl)propyl]-, [1S-[1R*(2S*, 3R*), 2S*]], and [1S-[1R*(2R*, 3S*), 2S*]].

### Part A:

To a solution of 863 mg (5.91 mmol) of meso-2,3-dimethylsuccinic acid in 7 mL of DMF at RT was added 1.13g (5.91 mmol) of EDC. After 15 minutes, a solution of 2.07g (5.91 mmol) of the product from Example 34, Part E and 1.4 mL of pyridine in 7 mL of anhydrous methylene chloride was added. After 11 hours, ethyl acetate was added and washed with 0.2N HCl, brine, dried and concentrated to afford 2.73g (97%) of a 1:1 mixture of diastereomeric acids.

### Part B:

To a solution of 1.45g (3.04 mmol) of the 1:1 mixture from Part A and 613 mg (4.51 mmol) of HOBT in 10 mL of DMF at 0°C was added 635 mg (3.31 mmol) of EDC. After 30 minutes at 0°C, 5 mL of conc. aqueous ammonia was added. After 1 hour at 0°C and 14 hours at RT, ethyl acetate was added, washed with 0.2N HCl, sat'd NaHCO₃, brine, dried and concentrated to afford 0.64g (44%) of a 1:1 mixture of amides.

These were separated on a Whatman 10 micron partisil column using 8%-14% isopropanol/-methylene chloride. The first isomer to elute was identified as butanediamide, N-[3-[[[(1,1-dimethylethyl)amino]carbonyl](3-methylbutyl)amino]-2-hydroxy-1-(phenylmethyl)propyl]-, [1S-[1R*(2R*, 3S*), 2S*], m/e/ = 477 (M + H).

The second isomer to elute was identified as butanediamide, N-[3-[[[(1,1-dimethylethyl)amino]-carbonyl](3-methylbutyl)amino]-2-hydroxy-1-(phenylmethyl)propyl]-, [1S-[1R*(2S*, 3R*), 2S*], m/e = 477 (M + H).

### Example 16

### Preparation of pentanediamide, N-[3-[[[(1,1-dimethylethyl)amino]carbonyl](3-methylbutyl)amino]-2-hydroxy-1-(phenylmethyl)propyl-3,3-dimethyl-, [1S-[1R*, 2S*].

### Part A:

To a solution of 232 mg (0.66 mmol) of the product from Example 7, Part E and 98 mg (1.2 mmol) of pyridine in 2 mL of methylene chloride was added 95 mg (0.66 mmol) of 3,3-dimethylglutaric anhydride at RT. After 15 hours, eth;yl acetate was added, washed with IN HCl, brine, dried and concentrated to afford 261 mg of crude product. Chromatography on silica gel using 5-20% methanol/methylene chloride afforded 108 mg of acid, m/e = 492 (M + H).

### Part B:

To a solution of 92 mg (0.19 mmol) of product from Part A and 38 mg (0.28 mmol) HOBT in 0.5 mL DMF at 0°C was added 36 mg (0.19 mmol) of EDC. After 30 minutes at 0°C, 0.25 mL of conc. aqueous ammonia was added. After 1 hour at 0°C and 16 hours at RT, ethyl acetate was added, washed with 0.2N HCl, sat'd NaHCO₃, brine, dried and concentrated to afford 72 mg of crude product. This was passed through a one-inch column of basic alumina with 10% methanol/methylene chloride to afford 53 mg of desired product, m/e = 491 (M + H).

### Example 17

### Preparation of butanediamide, N-[3-[[[(1,1-dimethylethyl)amino]carbonyl](3-methylbutyl)amino]-2-hydroxy-1-(phenylmethyl)propyl]-2,3-dimethyl-[1S-[1R*(2R*, 3S*), 2S*]](Isomer #1) and Preparation of butanediamide, N-[3-[[[(1,1-dimethylethyl)amino]carbonyl](3-methylbutyl)amino]-2-hydroxy-1-(phenylmethyl)propyl]-2,3-dimethyl-[1S-[1R*(2R*, 3S*), 2S*]] (Isomer #2).

### Part A:

To a solution of 1.47g (4.20 mmol) of the product from Example 7, Part E and 1.4 mL of pyridine in 9 mL of methylene chloride at RT was added 538 mg (4.20 mmol) of 2,2-dimethylsuccinic anhydride. After 15 hours, ethyl acetate was added and washed with 0.2N HCl, brine, dried and concentrated to afford 1.87g of crude product (approx. 3:1 mixture of isomers).

### Part B:

To a solution of 1.85g (3.9 mmol) of crude product from Part A and 887 mg (5.8 mmol) of HOBT in 10 mL of DMF at 0°C was added 809 mg (4.2 mmol) EDC. After 30 minutes at 0°C, 6 mL of conc. aqueous ammonia was added. After 1 hour at 0°C and 15 hours at RT, ethyl acetate was added, washed with 0.2N HCl, sat'd NaHCO₃, brine, dried and concentrated to afford 923 mg of crude product. The two isomers were separated on a Whatman Partisil 5 column using 8-14% isopropanol/methylene chloride. The major isomer was identified as Isomer #1, m/e = 477 (M + H).

The minor isomer was identified as Isomer #2, m/e = 477 (M + H).

### EXAMPLE 18

The compounds of the present invention are effective HIV protease inhibitors. Utilizing an enzyme assay as described below, the compounds set forth in the examples herein disclosed inhibited the HIV enzyme. The preferred compounds of the present invention and their calculated IC₅₀ (inhibiting concentration 50%, i.e., the concentration at which the inhibitor compound reduces enzyme activity by 50%) values are shown in Table 16, together with compounds os similar structure as claimed in divisional patent application EP 97105350.9 (publication number EP 813 867) and 97105351.7 (publication number EP 815 856). The enzyme method is described below. The substrate is 2-aminobenzoyl-Ile-Nle-Phe(p-NO₂)-Gln-ArgNH₂. The positive control is MVT-101 (Miller, M. et al, Science, 246, 1149 (1989)] The assay conditions are as follows:
- Assay buffer:: 20 mM sodium phosphate, pH 6.4
20% glycerol
1 mM EDTA
1 mM DTT
0.1% CHAPS

The above described substrate is dissolved in DMSO, then diluted 10 fold in assay buffer. Final substrate concentration in the assay is 80 µM.

HIV protease is diluted in the assay buffer to a final enzyme concentration of 12.3 nanomolar, based on a molecular weight of 10,780.

The final concentration of DMSO is 14% and the final concentration of glycerol is 18%. The test compound is dissolved in DMSO and diluted in DMSO to 10x the test concentration; 10µl of the enzyme preparation is added, the materials mixed and then the mixture is incubated at ambient temperature for 15 minutes. The enzyme reaction is initiated by the addition of 40µl of substrate. The increase in fluorescence is monitored at 4 time points (0, 8, 16 and 24 minutes) at ambient temperature. Each assay is carried out in duplicate wells.

**TABLE 3**

| Compound | | IC₅₀ (nanomolar) |
|---|---|---|
| 1a) | Butanediamide, N¹-[3-[[[(1,1-dimethylethyl)amino]carbonyl] (2-methylpropyl)amino]-2-hydroxy-1-(phenylmethyl)propyl]-2-[(N-benzyloxycarbonyl)amino]-, [1S-[1R*(R*), 2S*]]- | 35 |
| 1b) | Butanediamide, N¹-[3-[[[(1,1-dimethylethyl)amino]carbonyl] (2-methylpropyl)amino]-2-hydroxy-1-(phenylmethyl)propyl]-2-[(2-quinolinylcarbonyl)amino]-, [1S-[1R*(R*),2S*]]- | 7.1 |
| 2a) | Butanediamide, N¹-[3-[[[(1,1-dimethylethyl)amino]carbonyl](3-methylbutyl)amino]-2-hydroxy-1-(phenylmethyl)propyl]-2-[(N-benzyloxycarbonyl)amino], [1S-[1R*(R*),2S*]]- | 13.4 |
| 2b) | Butanediamide, N¹-[3-[[[(1,1-dimethylethyl)amino]carbonyl](3-methylbutyl)amino]-2-hydroxy-1-(phenylmethyl)propyl]-2-[(2-quinolinylcarbonyl)amino]-, [1S-[1R*(R*),2S*]]- | 3.2 |
| 3a) | Butanediamide, N¹-[3-[[[(1,1-dimethylethyl)amino]carbonyl](2-naphthylmethyl)amino]-2-hydroxy-1-(phenylmethyl)propyl]-2-[(N-benzyloxycarbonyl)amino]-, [1S-[1R*(R*), 2S*]]- | 31.0 |
| 3b) | Butanediamide, N¹-[3-[[[(1,1-dimethylethyl)amino]carbonyl](2-naphthylmethyl)amino]-2-hydroxy-1-(phenylmethyl)propyl]-2-[(2-quinolinylcarbonyl)amino]-, [1S-[1R*(R*),2S*]]- | 2.9 |
| 4a) | Butanediamide, N¹-[3-[[[(1,1-dimethylethyl)amino]carbonyl](2-phenylethyl)amino]-2-hydroxy-1-(phenylmethyl)propyl]-2-[(N-benzyloxycarbonyl)amino]-, [1S-[1R*(R*),2S*]]- | 70 |
| 4b) | Butanediamide, N¹-[3-[[[(1,1-dimethylethyl)amino]carbonyl] (2-phenylethyl)amino]-2-hydroxy-1-(phenylmethyl)propyl]-2-[(2-quinolinylcarbonyl)amino]-, [1S-[1R*(R*),2S*]]- | 10 |
| 5a) | Butanediamide, N¹-[3-[[[(1,1-dimethylethyl)amino]carbonyl](3,3-dimethylpropyl)amino]-2-hydroxy-1-(phenylmethyl)propyl]-2-[(N-benzyloxycarbonyl)amino], [1S-[1R*(R*),2S*]]- | 18 |
| 5b) | Butanediamide, N¹-[3-[[[(1,1-dimethylethyl)amino]carbonyl](3,3-dimethylpropyl)amino]-2-hydroxy-1-(phenylmethyl)propyl]-2-[(2-quinolinylcarbonyl)amino]-, [1S-[1R*(R*),2S*]]- | 3.7 |
| 6) | Propanamide, N-[3-[[[(1,1-dimethylethyl)amino]carbonyl] (3-methylbutyl)amino]-2-hydroxy-1-(phenylmethyl)propyl]-2-methyl-3-(methylsulfonyl)-, [1S-[1R*(R*), 2S*]]- | 29 |
| 7) | Propanamide, 3-(acetylamino)-N-[3-[[[(11,-dimethylethyl)amino]carbonyl] (3-methylbutyl)amino]-2-hydroxy-1-(phenylmethyl)propyl]-2-methyl-, [1S-[1R*(S*),2S*]]- | 100 |
| 8) | Propanamide, 3-(4-methoxybenzyloxycarbonyl)amino-N-[3-[[[(11,-dimethylethyl)amino]carbonyl](3-methylbutyl) amino]-2-hydroxy-1-(phenylmethyl) propyl]-2-methyl-, [1S-[1R*(S*),2S*]]- | 6 |
| 9) | Butanediamide, N¹-[2-hydroxy-3-[(4-fluorophenylmethyl)(1-pyrrolidinylcarbonyl)amino]-1-(phenylmethyl) propyl]-2-[(2-quinolinylcarbonyl) amino]-, [1S-[1R*(R*),2S*]]- | 7 |
| 10) | Butanediamide, N¹-[3-[[[(1,1-dimethylethyl)amino]carbonyl](3-methylbutyl)amino]-2-hydroxy-1-(phenylmethyl)propyl]-2,3,3-trimethyl-, [1S-[1R*(2S*),2S*]]- | 12 |
| 11) | Butanoic acid, 4-[[3-[[[(1,1-dimethylethyl)amino]carbonyl] (3-methylbutyl)amino]-2-hydroxy-1-(phenylmethyl)propyl]amino-2,2, 3-trimethyl-4-oxo-, [1S-[1R*(3S*), 2S*]]- | 8 |
| 12) | Butaneamide,2-(acetylamino)-N-[3-[[[(1,1-dimethylethyl)amino]carbonyl](3-methylbutyl)amino]-2-hydroxy-1-(phenylmethyl)propyl]-3,3-dimethyl-, [1S-[1R*(R*),2S*]]- | 18 |

Utilizing the procedures set forth above in the examples along with the general description, it is contemplated that the compounds listed below could be prepared and that such compounds would have activities as HIV protease inhibitors substantially similar to the activities of the compounds set forth in the examples.
Butanediamide, N¹-[3-[[[(1,1-dimethylethyl)amino]carbonyl]pentylamino]-2-hydroxy-1-(phenylmethyl)propyl]-2-[(2-quinolinylcarbonyl)amino]-, [1S-[1R*(R*),2S*]]-
Butanediamide, N¹-[3-[[[(1,1-dimethylethyl)amino]carbonyl]hexylamino]-2-hydroxy-1-(phenylmethyl)propyl]-2-[(2-quinolinylcarbonyl)amino]-, [1S-[1R*(R*),2S*]]-
Propanamide, N-[3-[[[(1,1-dimethylethyl)amino]carbonyl]butylamino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](phenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](4-fluorophenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](4-methoxyphenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](cyclohexylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](3-methylbutyl)amino]-2-hydroxy-1-(cyclohexylmethyl)propyl]-2-methyl-3-(methylsulfonyl)-, [1S-[1R*(R*), 2S*]]-
Butanediamide, N¹-[2-hydroxy-3-[(3-methylbutyl)(1-pyrrolidinylcarbonyl)amino)-1-(phenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-pyrrolidinylcarbonyl)amino]-1-(4-fluorophenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-pyrrolidinylcarbonyl)amino]-1-(cyclohexylmethyl)propyl]-2-[(2-quinolinylcarbonyl)amino]-, [1S-[1R*(R*),2S*]]-
Butanediamide, N¹-[2-hydroxy-3-[(phenylmethyl)(4-morpholinylcarbonyl)amino]-1-(phenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-piperidinylcarbonyl)amino]-1-(phenylmethyl)propyl]-2-[(2-quinolinylcarbonyl)amino]-, [1S-[1R*(R*),2S*]]-
Butanediamide, N¹-[2-hydroxy-3-[(phenylmethyl)(1-piperidinylcarbonyl)amino]-1-(phenylmethyl)propyl]-2-[(2-quinolinylcarbonyl)amino]-, [1S-[1R*(R*),2S*]]-
Butanediamide, N-[3-[[[(1,1-dimethylethyl)amino]carbonyl]butylamino]-2-hydroxy-1-(phenylmethyl)propyl]-2,3,3-trimethyl-, [1S-[1R*(2S*),2S*]]-
Butanediamide, N-[3-[[[(1,1-dimethylethyl)amino]carbonyl](phenylmethyl)amino]-2-hydroxy-1-(phenylmethyl)propyl]-2,3,3-trimethyl-, [1S-[1R*(2S*),2S*]]-
Butanediamide, N-[3-[[[(1,1-dimethylethyl)amino]carbonyl](4-fluorophenylmethyl)amino]-2-hydroxy-1-(phenylmethyl)propyl]-2,3,3-trimethyl-, [1S-[1R*(2S*),2S*]]-
Butanediamide, N-[3-[[[(1,1-dimethylethyl)amino]carbonyl](cyclohexylmethyl)amino]-2-hydroxy-1-(phenylmethyl)propyl]-2,3,3-trimethyl-, [1S-[1R*(2S*),2S*]]-
Butanediamide, N-[3-[[[(1,1-dimethylethyl)amino]carbonyl](3-methylbutyl)amino]-2-hydroxy-1-(cyclohexylmethyl)propyl]-2,3,3-trimethyl-, [1S-[1R*(2S*),2S*]]-
Butanoic acid, 4-[[3-[[[(1,1-dimethylethyl)amino]carbonyl]butylamino]-2-hydroxy-1-(phenylmethyl)propyl]amino-2,2,3-trimethyl-4-oxo-, [1S-[1R*(3S*),2S*]]-
Butanoic acid, 4-[[3-[[[(1,1-dimethylethyl)amino]carbonyl](phenylmethyl)amino]-2-hydroxy-1-(phenylmethyl)propyl]amino-2,2,3-trimethyl-4-oxo-, [1S-[1R*(3S*),2S*]]-
Butanoic acid, 4-[[3-[[[(1,1-dimethylethyl)amino]carbonyl](4-fluorophenylmethyl)amino]-2-hydroxy-1-(phenylmethyl)propyl]amino-2,2,3-trimethyl-4-oxo-, [1S-[1R*(3S*),2S*]]-
Butanoic acid, 4-[[3-[[[(1,1-dimethylethyl)amino]carbonyl](cyclohexylmethyl)amino]-2-hydroxy-1-(phenylmethyl)propyl]amino-2,2,3-trimethyl-4-oxo-, [1S-[1R*(3S*),2S*]]-
Butaneamide, 2-(acetylamino)-N-[3-[[[(1,1-dimethylethyl)amino]carbonyl](3-methylbutyl)amino]-2-hydroxy-1-(cyclohexylmethyl)propyl]-3,3-dimethyl-, [1S-[1R*(R*),2S*]]-
Butaneamide, 2-(acetylamino)-N-[3-[[[(1,1-dimethylethyl)amino]carbonyl]butylamino]-2-hydroxy-1-(phenylmethyl)propyl]-3,3-dimethyl-, [1S-[1R*(R*),2S*]]-
Butaneamide, 2-(acetylamino)-N-[3-[[[(1,1-dimethylethyl)amino]carbonyl](phenylmethyl)amino]-2-hydroxy-1-(phenylmethyl)propyl]-3,3-dimethyl-, [1S-[1R*(R*),2S*]]-
Butaneamide, 2-(acetylamino)-N-[3-[[[(1,1-dimethylethyl)amino]carbonyl](cyclohexylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](3-methylbutyl)amino]-2-hydroxy-1-(4-fluorophenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](3-methylbutyl)amino]-2-hydroxy-1-(cyclohexylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl]butylamino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](phenylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](4-fluorophenylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](cyclohexylmethyl)amino]-2-hydroxy-1-(phenylmethyl)propyl]-3,3-dimethyl-, [1S-[1R*(R*),2S*]]-

The compounds of the present invention are effective antiviral compounds and, in particular, are effective retroviral inhibitors as shown above. Thus, the subject compounds are effective HIV protease inhibitors. It is contemplated that the subject compounds will also inhibit other viruses such as HIV, human T-cell leukemia virus, respiratory syncitial virus, hepadnavirus, cytomegalovirus and picornavirus.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

The foregoing is merely illustrative of the invention and is not intended to limit the invention to the disclosed compounds.

The preceding examples can be repeated with similar success by substituting the generically or specifically described reactants and/or operating conditions of this invention for those used in the preceding examples.

## Claims

1. Use of a compound represented by the formula: 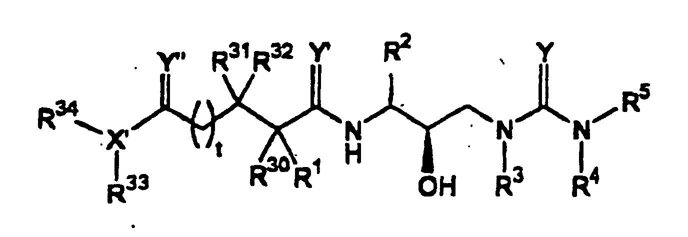 for preparing a medicament for inhibiting a retroviral protease wherein:
R¹ represents hydrogen, -CH₂SO₂NH₂, alkyl and cycloalkyl radicals and amino acid side chains selected from the group consisting of asparagine, S-methyl cysteine and the corresponding sulfoxide and sulfone derivatives thereof, glycine, allo-isoleucine, alanine, leucine, tert-leucine, phenylalanine, ornithine, threonine, allo-threonine, isoleucine, histidine, norleucine, valine, glutamine, serine, aspartic acid and betacyano alanine side chains;
R² represents alkyl, aryl, cycloalkyl, cycloalkylalkyl and aralkyl radicals optionally substituted with a group selected from -OR⁹, -SR⁹, and halogen radicals wherein R⁹ represents hydrogen and alkyl radicals;
R³ represents alkyl, alkenyl, hydroxyalkyl, cycloalkyl, cycloalkylalkyl, heterocycloalkyl,
heterocycloalkylalkyl, aryl, heteroaryl, aralkyl, and heteroaralkyl radicals;
R⁴ and R⁵ independently represent hydrogen and radicals as defined by R³, or R⁴ and R⁵, together with the nitrogen. atom to which they are bonded represent heterocycloalkyl and heteroaryl radicals;
R³⁰, R³¹ and R³² represent radicals as defined for R¹, or one of R¹ and R³⁰ together with one of R³¹ and R³² and the carbon atoms to which they are attached form a cycloalkyl radical;
R³³ and R³⁴ independently represent hydrogen and radicals as defined for R³, or R³³ and R³⁴ together with X' represent cycloalkyl, aryl, heterocyclyl and heteroaryl radicals, provided that when X' is O, R³⁴ is absent;
X' represents N, O or C(R¹⁷) wherein R¹⁷ represents hydrogen and alkyl radicals, Y, Y' and Y" independently represent 0 and S, t represents 0 or 1; and
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro,
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

2. The use recited in claim 1 wherein Y, Y' and Y" are O.

3. The use recited in claim 1 wherein t is 0.

4. The use recited in claim 1 wherein X' represents N.

5. The use recited in claim 1 wherein R₁ represents hydrogen and alkyl radicals having from 1 to about 4 carbon atoms, aralkyl radicals, hydroxyl radicals, and radicals represented by the formula CH₂C(O)R" wherein R" represents -R³⁸, and -NR³⁸R³⁹ wherein R³⁸ and R³⁹ independently represent hydrogen and alkyl radicals having from 1 to about 4 carbon atoms.

6. The use recited in claim 1 wherein R¹ represents hydrogen, methyl, ethyl, benzyl, phenylpropyl, hydroxyl and radicals represented by the formula -CH₂C(O)R" wherein R" represents -CH₃, -NH₂, and -OH.

7. The use recited in claim 1 wherein X' is O, R³⁴ is absent and R³³ represents hydrogen, an alkyl radical or an aralkyl radical.

8. The use recited in claim 1 wherein R² represents alkyl, cycloalkylalkyl and aralkyl radicals, which radicals are optionally substituted with halogen radicals and radicals represented by the f ormula -OR⁹ and -SR⁹ wherein R⁹ represents alkyl radicals.

9. The use recited in claim 1 wherein R² represents CH₃SCH₂CH₂-, iso-butyl, n-butyl, benzyl, 2-naphthylmethyl and cyclohexylmethyl radicals.

10. The use recited in claim 1 wherein R² represents benzyl, 4-fluorobenzyl, and 2-naphthylmethyl radicals.

11. The use recited in claim 1 wherein R³, R⁴ and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals.

12. The use recited in claim 1 wherein R³ represents benzyl, para-fluorobenzyl, paramethoxybenzyl, para-methylbenzyl, and 2-naphthylmethyl radicals and R⁴ represents t-butyl.

13. The use recited in claim 1 wherein R⁴ and R⁵ together with the nitrogen to which they are bonded represent pyrrolidinyl, piperidinyl, morpholinyl, and piperazinyl radicals.

14. The use recited in claim 1 wherein the retroviral protease is HIV protease.

15. Use of a compound represented by the formula: 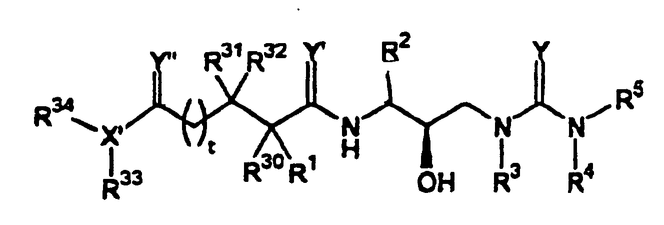 for preparing a medicament for treating a retroviral infection wherein:
R¹ represents hydrogen, -CH₂SO₂NH₂, alkyl and cycloalkyl radicals and amino acid side chains selected from the group consisting of asparagine, S-methyl cysteine and the corresponding sulfoxide and sulfone derivatives thereof, glycine, allo-isoleucine, alanine, leucine, tert-leucine, phenylalanine, ornithine, threonine, allo-threonine, isoleucine, histidine, norleucine, valine, glutamine, serine, aspartic acid and betacyano alanine side chains;
R² represents alkyl, aryl, cycloalkyl, cycloalkylalkyl and aralkyl radicals optionally substituted with a group selected from -OR⁹, -SR⁹, and halogen radicals wherein R⁹ represents hydrogen and alkyl radicals;
R³ represents alkyl, alkenyi, hydroxyalkyl, cycloalkyl, cycloalkylalkyl, heterocycloalkyl,
heterocycloalkylalkyl, aryl, heteroaryl, aralkyl, and heteroaralkyl radicals;
R⁴ and R⁵ independently represent hydrogen and radicals as defined by R³, or R⁴ and R⁵, together with the nitrogen, atom to which they are bonded represent heterocycloalkyl and heteroaryl radicals;
R³⁰, R³¹ and R³² represent radicals as defined for R¹, or one of R¹ and R³⁰ together with one of R³¹ and R³² and the carbon atoms to which they are attached form a cycloalkyl radical;
R³³ and R³⁴ independently represent hydrogen and radicals as defined for R³, or R³³ and R³⁴ together with X' represent cycloalkyl, aryl, heterocyclyl and heteroaryl radicals, provided that when X' is O, R³⁴ is absent;
X' represents N, O or C(R¹⁷) wherein R¹⁷ represents hydrogen and alkyl radicals, Y, Y' and Y" independently represent O and S.
t represents 0 or 1; and
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro,
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

16. The use recited in claim 15 wherein Y, Y' and Y" are O.

17. The use recited in claim 15 wherein t is 0.

18. The use recited in claim 15 wherein X' represents N.

19. The use recited in claim 15 wherein R₁ represents hydrogen and alkyl radicals having from 1 to about 4 carbon atoms, aralkyl radicals, hydroxyl radicals, and radicals represented by the formula CH₂C(O)R" wherein R" represents -R³⁸, and -NR³⁸R³⁹ wherein R³⁸ and R³⁹ independently represent hydrogen and alkyl radicals having from 1 to about 4 carbon atoms.

20. The use recited in claim 15 wherein R¹ represents hydrogen, methyl, ethyl, benzyl, phenylpropyl, hydroxyl and radicals represented by the formula -CH₂C(O)R" wherein R" represents -CH₃, -NH₂, and -OH.

21. The use recited in claim 15 wherein X' is O, R³⁴ is absent and R³³ represents hydrogen, an alkyl radical or an aralkyl radical.

22. The use recited in claim 15 wherein R² represents alkyl, cycloalkylalkyl and aralkyl radicals, which radicals are optionally substituted with halogen radicals and radicals represented by the formula -OR⁹ and -SR⁹ wherein R⁹ represents alkyl radicals.

23. The use recited in claim 15 wherein R² represents CH₃SCH₂CH₂-, iso-butyl, n-butyl, benzyl, 2-naphthylmethyl and cyclohexylmethyl radicals.

24. The use recited in claim 15 wherein R² represents benzyl, 4-fluorobenzyl, and 2-naphthylmethyl radicals.

25. The use recited in claim 15 wherein R³, R⁴ and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals.

26. The use recited in claim 15 wherein R³ represents benzyl, para-fluorobenzyl, paramethoxybenzyl, para-methylbenzyl, and 2-naphthylmethyl radicals and R⁴ represents t-butyl.

27. The use recited in claim 15 wherein R⁴ and R⁵ together with the nitrogen to which they are bonded represent pyrrolidinyl, piperidinyl, morpholinyl, and piperazinyl radicals.

28. The use recited in claim 15 wherein the retroviral infection is an HIV infection.

29. Use of a compound represented by the formula: 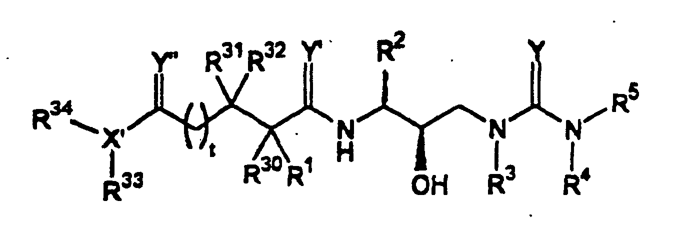 for preparing a medicament for treating AIDS wherein:
R¹ represents hydrogen, -CH₂SO₂NH₂, alkyl and cycloalkyl radicals and amino acid side chains selected from the group consisting of asparagine, S-methyl cysteine and the corresponding sulfoxide and sulfone derivatives thereof, glycine, allo-isoleucine, alanine, leucine, tert-leucine, phenylalanine, ornithine, threonine, allo-threonine, isoleucine, histidine, norleucine, valine, glutamine, serine, aspartic acid and betacyano alanine side chains;
R² represents alkyl, aryl, cycloalkyl, cycloalkylalkyl and aralkyl radicals optionally substituted with a group selected from -OR⁹, -SR⁹, and halogen radicals wherein R⁹ represents hydrogen and alkyl radicals;
R³ represents alkyl, alkenyi, hydroxyalkyl, cycloalkyl, cycloalkylalkyl, heterocycloalkyl,
heterocycloalkylalkyl, aryl, heteroaryl, aralkyl, and heteroaralkyl radicals;
R⁴ and R⁵ independently represent hydrogen and radicals as defined by R³, or R⁴ and R⁵, together with the nitrogen. atom to which they are bonded represent heterocycloalkyl and heteroaryl radicals;
R³⁰, R³¹ and R³² represent radicals as defined for R¹, or one of R¹ and R³⁰ together with one of R³¹ and R³² and the carbon atoms to which they are attached form a cycloalkyl radical;
R³³ and R³⁴ independently represent hydrogen and radicals as defined for R³, or R³³ and R³⁴ together with X' represent cycloalkyl, aryl, heterocyclyl and heteroaryl radicals, provided that when X' is O, R³⁴ is absent;
X' represents N, O or C(R¹⁷) wherein R¹⁷ represents hydrogen and alkyl radicals, Y, Y' and Y" independently represent O and S.
t represents 0 or 1; and
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms
in which one hydrogen is replaced by an aryl,
- aryl is phenyl or naphthyl, which optionally carries one or more substituents selected from alkyl, alkoxy, halogen, hydroxy, amino and nitro,
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

30. The use recited in claim 29 wherein Y, Y' and Y" are O.

31. The use recited in claim 29 wherein t is 0.

32. The use recited in claim 29 wherein X' represents N.

33. The use recited in claim 29 wherein R₁ represents hydrogen and alkyl radicals having from 1 to about 4 carbon atoms, aralkyl radicals, hydroxyl radicals, and radicals represented by the formula CH₂C(O)R" wherein R" represents -R³⁸, and -NR³⁸R³⁹ wherein R³⁸ and R³⁹ independently represent hydrogen and alkyl radicals having from 1 to about 4 carbon atoms.

34. The use recited in claim 33 wherein R¹ represents hydrogen, methyl, ethyl, benzyl, phenylpropyl, hydroxyl and radicals represented by the formula -CH₂C(O)R" wherein R" represents -CH₃, -NH₂, and -OH.

35. The use recited in claim 29 wherein X' is O, R³⁴ is absent and R³³ represents hydrogen, an alkyl radical or an aralkyl radical.

36. The use recited in claim 29 wherein R² represents alkyl, cycloalkylalkyl and aralkyl radicals, which radicals are optionally substituted with halogen radicals and radicals represented by the f ormula -OR⁹ and -SR⁹ wherein R⁹ represents alkyl radicals.

37. The use recited in claim 29 wherein R² represents CH₃SCH₂CH₂-, iso-butyl, n-butyl, benzyl, 2-naphthylmethyl and cyclohexylmethyl radicals.

38. The use recited in claim 29 wherein R² represents benzyl, 4-fluorobenzyl, and 2-naphthylmethyl radicals.

39. The use recited in claim 29 wherein R³, R⁴ and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals.

40. The use recited in claim 29 wherein R³ represents benzyl, para-fluorobenzyl, paramethoxybenzyl, para-methylbenzyl, and 2-naphthylmethyl radicals and R⁴ represents t-butyl.

41. The use recited in claim 29 wherein R⁴ and R⁵ together with the nitrogen to which they are bonded represent pyrrolidinyl, piperidinyl, morpholinyl, and piperazinyl radicals.

42. The use recited in any of the claims 1, 15, 29 wherein the compound is selected among the group consisting of compounds of the following general formula 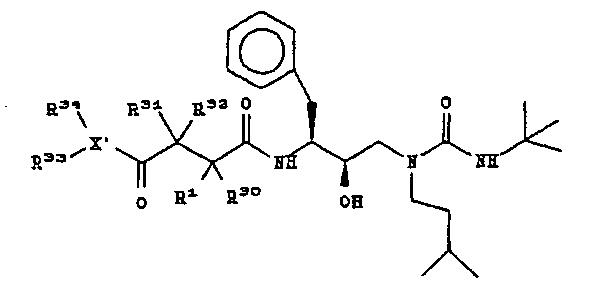 wherein the substituents are as listed in the following Table
| R¹ | R³⁰ | R³¹ | R³² | X' | R³³ | R³⁴ |
|---|---|---|---|---|---|---|
| H | H | H | H | N | H | H |
| H | H | H | H | O | H | - |
| H | H | H | H | O | CH₃ | - |
| | | | | | | |
| CH₃ | H | H | H | N | H | H |
| CH₃ | H | H | H | O | H | - |
| | | | | | | |
| H | H | CH₃ | H | N | H | H |
| H | H | CH₃ | H | O | H | - |
| | | | | | | |
| CH₃ | CH₃ | H | H | N | H | H |
| CH₃ | CH₃ | H | H | O | H | - |
| CH₃ | CH₃ | H | H | O | CH₂C₆H₄OCH₃ | - |
| | | | | | | |
| H | H | CH₃ | CH₃ | N | H | H |
| H | H | CH₃ | CH₃ | O | H | - |
| H | H | CH₃ | CH₃ | O | CH₂C₆H₄OCH₃ | - |
| CH₃ | H | CH₃ | H | N | H | H |
| CH₃ | H | CH₃ | H | N | H | CH₃ |
| CH₃ | H | CH₃ | H | N | CH₃ | CH₃ |
| CH₃ | H | CH₃ | H | O | H | - |
| CH₃ | H | CH₃ | H | N | H | -CH₂C₆H₅OCH₃ |
| OH | H | H | H | N | H | H |
| OH | H | H | H | O | H | - |
| H | H | OH | H | N | H | H |
| H | H | OH | H | O | H | - |
| CH₂ | H | H | H | N | H | H |
| | | | | | | |
| CH₂C(O)NH₂ | H | H | H | N | H | H |
| | | | | | | |
| CH₂C(O)NH₂ | H | H | H | O | H | - |
| | | | | | | |
| CH₂C(O)NH₂ | H | H | H | O | CH₃ | - |
| | | | | | | |
| CH₂Ph | H | H | H | N | H | H |

43. The use recited in any of the claims 1, 15, 29 wherein the compound is selected among the group consisting of compounds of the formulas

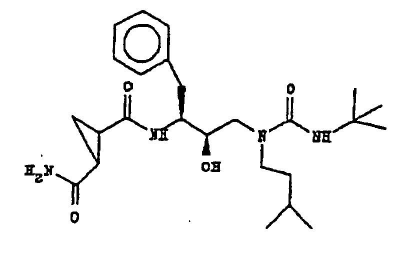

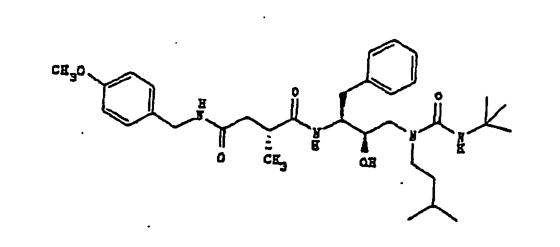

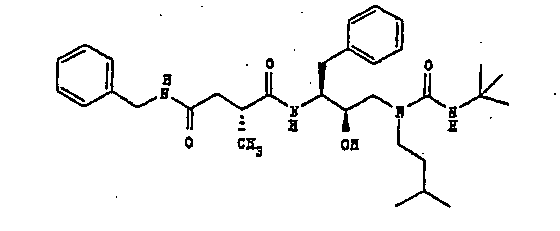

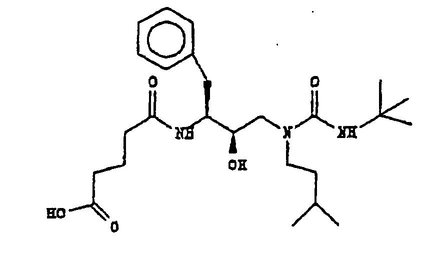

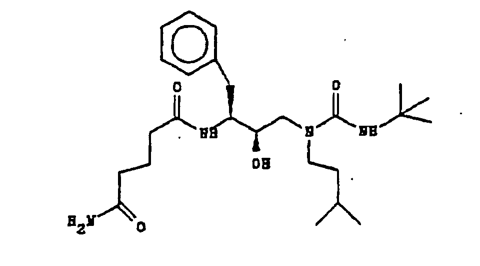

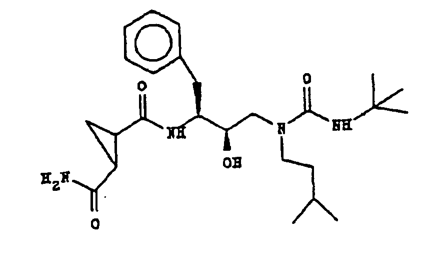

44. The use recited in any one of the claims 1, 15 and 29 wherein the compound is selected among the group consisting of
Butanoic acid, 4-[[3-[[[(1,1-dimethylethyl)amino]carbonyl]-butylamino]-2-hydroxy-1-(phenylmethyl)propyl]amino- 2,2,3-trimethyl-4-oxo-, [1S-[1R*(3S*),2S*]]-
Butanoic acid, 4-[[3-[[[(1,1-dimethylethyl)amino]carbonyl]-(phenylmethyl)amino]-2-hydroxy-1-(phenylmethyl)propyl]-amino-2,2,3-trimethyl-4-oxo-, [1S-[1R*(3S*),2S*]]-
Butanoic acid, 4-[(3-[[[(1,1-dimethylethyl)amino]carbonyl](4-fluorophenylmethyl) amino]-2-hydroxy-1-(phenylmethyl)propyl]amino-2,2,3-trimethyl-4-oxo -, [1S-[1R*(3S*),2S*]]-
Butanoic acid, 4-[[3-[[[(1,1-dimethylethyl)amino]carbonyl](cyclohexylmethyl) amino]-2-hydroxy-1-(phenylmethyl)propyl]amino-2,2,3-trimethyl-4-oxo-, [1S-[1R*(3S*),2S*]]-
Butanediamide, N-[3-[[[(1,1-dimethylethyl)amino]carbonyl](3-methylbutyl)amino]-2-hydroxy-1-(phenylmethyl)propyl]-2,3-dimethyl-[1S-[1R*(2R*, 3S*), 2S*]]
Butanoic acid, 4-[3-[[[(1,1-dimethylethyl)amino]carbonyl] (3-methylbutyl)amino]-2-hydroxy-1-(phenylmethyl)propyl]amino-2,2,3-trimethyl-4-oxo-, [1S-[1R*(3S*),2S*]]-

## Patentansprüche

1. Verwendung einer Verbindung mit der Formel 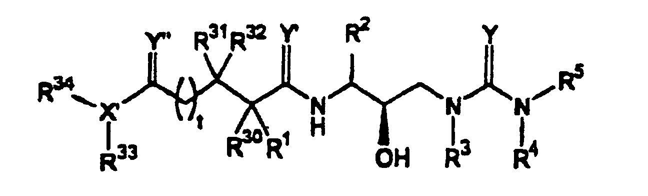 für die Herstellung eines Arzneimittels zum Inhibieren einer Retrovirusprotease, worin
- R¹ Wasserstoff, -CH₂SO₂NH₂, einen Alkyl- und Cycloalkylrest und eine Aminosäureseitenkette, die aus der Gruppe ausgewählt ist, die aus Asparagin, S-Methylcystein und deren entsprechenden Sulfoxid- und Sulfonderivaten, einer Glycin-, Alloisoleucin-, Alanin-, Leucin-, tert.-Leucin-, Phenylalanin-, Ornithin-, Threonin-, Allothreonin-, Isoleucin-, Histidin-, Norleucin-, Valin-, Glutamin-, Serin-, Asparaginsäure- und β-Cyanoalaninseitenkette besteht,
- R² einen Alkyl-, Aryl-, Cycloalkyl-, Cycloalkylalkyl- und Aralkylrest, der wahlweise mit einer Gruppe substituiert ist, die aus -OR⁹, -SR⁹ und einem Halogenrest ausgewählt ist, wobei R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Heteroaryl-, Aralkyl- und Heteroalkylrest bedeutet und
- R⁴ und R⁵ unabhängig voneinander Wasserstoff und einen wie für R³ definierten Rest oder R⁴ und R⁵ zusammen mit dem Stickstoffatom, an welches sie gebunden sind, einen Heterocycloalkyl- und Heteroarylrest bedeuten,
- R³⁰, R³¹ und R³² jeweils einen wie für R¹ definierten Rest oder einer von R¹ und R³⁰ zusammen mit einem von R³¹ und R³² und den Kohlenstoffatomen, an welche sie gebunden sind, einen Cycloalkylrest bilden, und
- R³³ und R³⁴ unabhängig voneinander Wasserstoff und einen wie für R³ definierten Rest oder R³³ und R³⁴ zusammen mit X' einen Cycloalkyl-, Aryl-, Heterocyclyl- und Heteroarylrest, vorausgesetzt, dass, wenn X' O bedeutet, R³⁴ fehlt, bedeuten,
- X' N, O oder C(R¹⁷) bedeutet, worin R¹⁷ Wasserstoff und einen Alkylrest bedeutet, und Y, Y' und Y" unabhängig voneinander 0 und S bedeuten und
- t 0 oder 1 bedeutet, worin
• ein Alkylrest einen Alkylrest mit 1 bis 10 Kohlenstoffatomen,
• ein Cycloalkylrest einen Cycloalkylrest mit 3 bis 8 Kohlenstoffatomen,
• ein Aralkylrest einen Alkylrest mit 1 bis 10 Kohlenstoffatomen, in welchem ein Wasserstoffatom durch einen Arylrest ersetzt ist,
• ein Arylrest einen Phenyl- oder Naphthylrest, der wahlweise einen oder mehrere Substituenten trägt, die aus Alkyl, Alkoxy, Halogen, Hydroxy, Amino und Nitro ausgewählt sind,
• ein Heterocycloalkylrest einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, der wahlweise an einem oder mehreren Kohlenstoffatomen mit Halogen, Alkyl, Alkoxy oder Oxo und/oder an einem sekundären Stickstoffatom mit Alkyl, Aralkoxycarbonyl, Alkanoyl, Phenyl oder Phenylalkyl oder an einem tertiären Stickstoffatom mit Oxido substituiert ist und welcher über ein Kohlenstoffatom gebunden ist,
• ein Heteroarylrest einen aromatischen monocyclischen, bicyclischen oder tricyclischen Heterocyclus, der die Heteroatome enthält und wahlweise wie zuvor für den Heterocycloalkylrest definiert substituiert ist, und
• ein Heteroaralkylrest einen Alkylrest mit 1 bis 10 Kohlenstoffatomen, in welchem ein Wasserstoffatom durch einen Heteroarylrest ersetzt ist,
bedeutet.

2. Verwendung nach Anspruch 1, wobei Y, Y' und Y" O bedeuten.

3. Verwendung nach Anspruch 1, wobei t 0 bedeutet.

4. Verwendung nach Anspruch 1, wobei X' N bedeutet.

5. Verwendung nach Anspruch 1, wobei R, Wasserstoff und einen Alkylrest mit 1 bis etwa 4 Kohlenstoffatomen, Aralkyl- und Hydroxylrest und einen Rest mit der Formel CH₂C(O)R", worin R" -R³⁸ bedeutet, und -NR³⁸R³⁹, worin R³⁸ und R³⁹ unabhängig voneinander Wasserstoff und einen Alkylrest mit 1 bis etwa 4 Kohlenstoffatomen bedeuten, bedeutet.

6. Verwendung nach Anspruch 1, wobei R¹ einen Wasserstoff-, Methyl-, Ethyl-, Benzyl-, Phenylpropyl-und Hydroxylrest und einen Rest mit der Formel -CH₂C(O)R" bedeutet, worin R" -CH₃, -NH₂ und -OH bedeutet.

7. Verwendung nach Anspruch 1, wobei X' O bedeutet, R³⁴ fehlt und R³³ einen Wasserstoff-, Alkyl- oder Aralkylrest bedeutet.

8. Verwendung nach Anspruch 1, wobei R² einen Alkyl-, Cycloalkylalkyl- und Aralkylrest bedeutet, wobei die Reste wahlweise mit Halogenresten und Resten mit der Formel -OR⁹ und -SR⁹, worin R⁹ einen Alkylrest bedeutet, substituiert sind.

9. Verwendung nach Anspruch 1, wobei R² CH₃SCH₂CH₂-, einen Isobutyl-, n-Butyl-, Benzyl-, 2-Naphthylmethyl- und Cyclohexylmethylrest bedeutet.

10. Verwendung nach Anspruch 1, wobei R² einen Benzyl-, 4-Fluorbenzyl- und 2-Naphthylmethylrest bedeutet.

11. Verwendung nach Anspruch 1, wobei R³, R⁴ und R⁵ unabhängig voneinander einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- und Heteroaralkylrest bedeuten.

12. Verwendung nach Anspruch 1, wobei R³ einen Benzyl-, p-Fluorbenzyl-, p-Methoxybenzyl-, p-Methylbenzyl- und 2-Naphthylmethylrest und R⁴ einen *tert*.-Butylrest bedeutet.

13. Verwendung nach Anspruch 1, wobei R⁴ und R⁵ zusammen mit dem Stickstoffatom, an welches sie gebunden sind, einen Pyrrolidinyl-, Piperidinyl-, Morpholinyl- und Piperazinylrest bedeuten.

14. Verwendung nach Anspruch 1, wobei die Retrovirusprotease HIV-Protease ist.

15. Verwendung einer Verbindung mit der Formel 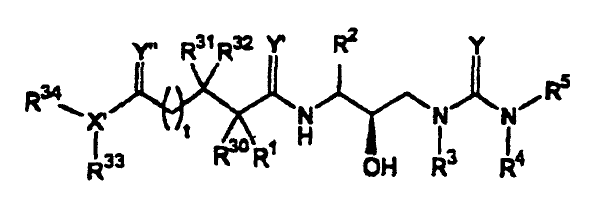 für die Herstellung eines Arzneimittels zur Behandlung einer Retrovirusinfektion, worin
- R¹ Wasserstoff, -CH₂SO₂NH₂, einen Alkyl- und Cycloalkylrest und eine Aminosäureseitenkette, die aus der Gruppe ausgewählt ist, die aus Asparagin, S-Methylcystein und deren entsprechenden Sulfoxid- und Suffonderivaten, einer Glycin-, Alloisoleucin-, Alanin-, Leucin-, *tert*.-Leucin-, Phenylalanin-, Ornithin-, Threonin-, Allothreonin-, Isoleucin-, Histidin-, Norleucin-, Valin-, Glutamin-, Serin-, Asparaginsäure- und β-Cyanoalaninseitenkette besteht,
- R² einen Alkyl-, Aryl-, Cycloalkyl-, Cycloalkylalkyl- und Aralkylrest, der wahlweise mit einer Gruppe substituiert ist, die aus -OR⁹, -SR⁹ und einem Halogenrest ausgewählt ist, wobei R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Heteroaryl-, Aralkyl- und Heteroalkylrest bedeutet und
- R⁴ und R⁵ unabhängig voneinander Wasserstoff und einen wie für R³ definierten Rest oder R⁴ und R⁵ zusammen mit dem Stickstoffatom, an welches sie gebunden sind, einen Heterocycloalkyl- und Heteroarylrest bedeuten,
- R³⁰, R³¹ und R³² jeweils einen wie für R¹ definierten Rest oder einer von R¹ und R³⁰ zusammen mit einem von R³¹ und R³² und den Kohlenstoffatomen, an welche sie gebunden sind, einen Cycloalkylrest bilden, und
- R³³ und R³⁴ unabhängig voneinander Wasserstoff und einen wie für R³ definierten Rest oder R³³ und R³⁴ zusammen mit X' einen Cycloalkyl-, Aryl-, Heterocyclyl- und Heteroarylrest, vorausgesetzt, dass, wenn X' O bedeutet, R³⁴ fehlt, bedeuten,
- X' N, O oder C(R¹⁷) bedeutet, worin R¹⁷ Wasserstoff und einen Alkylrest bedeutet, und Y, Y' und Y" unabhängig voneinander O und S bedeuten und
- t 0 oder 1 bedeutet, worin
• ein Alkylrest einen Alkylrest mit 1 bis 10 Kohlenstoffatomen,
• ein Cycloalkylrest einen Cycloalkylrest mit 3 bis 8 Kohlenstoffatomen,
• ein Aralkylrest einen Alkylrest mit 1 bis 10 Kohlenstoffatomen, in welchem ein Wasserstoffatom durch einen Arylrest ersetzt ist,
• ein Arylrest einen Phenyl- oder Naphthylrest, der wahlweise einen oder mehrere Substituenten trägt, die aus Alkyl, Alkoxy, Halogen, Hydroxy, Amino und Nitro ausgewählt sind,
• ein Heterocycloalkylrest einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, der wahlweise an einem oder mehreren Kohlenstoffatomen mit Halogen, Alkyl, Alkoxy oder Oxo und/oder an einem sekundären Stickstoffatom mit Alkyl, Aralkoxycarbonyl, Alkanoyl, Phenyl oder Phenylalkyl oder an einem tertiären Stickstoffatom mit Oxido substituiert ist und welcher über ein Kohlenstoffatom gebunden ist,
• ein Heteroarylrest einen aromatischen monocyclischen, bicyclischen oder tricyclischen Heterocyclus, der die Heteroatome enthält und wahlweise wie zuvor für den Heterocycloalkylrest definiert substituiert ist, und
• ein Heteroaralkylrest einen Alkylrest mit 1 bis 10 Kohlenstoffatomen, in welchem ein Wasserstoffatom durch einen Heteroarylrest ersetzt ist,
bedeutet.

16. Verwendung nach Anspruch 15, wobei Y, Y' und Y" O bedeuten.

17. Verwendung nach Anspruch 15, wobei t 0 bedeutet.

18. Verwendung nach Anspruch 15, wobei X' N bedeutet.

19. Verwendung nach Anspruch 15, wobei R, Wasserstoff und einen Alkylrest mit 1 bis etwa 4 Kohlenstoffatomen, Aralkyl- und Hydroxylrest und einen Rest mit der Formel CH₂C(O)R", worin R" -R³⁸ bedeutet, und -NR³⁸R³⁹, worin R³⁸ und R³⁹ unabhängig voneinander Wasserstoff und einen Alkylrest mit 1 bis etwa 4 Kohlenstoffatomen bedeuten, bedeutet.

20. Verwendung nach Anspruch 15, wobei R¹ einen Wasserstoff-, Methyl-, Ethyl-, Benzyl-, Phenylpropyl-und Hydroxylrest und einen Rest mit der Formel -CH₂C(O)R" bedeutet, worin R" -CH₃, -NH₂ und -OH bedeutet.

21. Verwendung nach Anspruch 15, wobei X' O bedeutet, R³⁴ fehlt und R³³ einen Wasserstoff-, Alkyl- oder Aralkylrest bedeutet.

22. Verwendung nach Anspruch 1, wobei R² einen Alkyl-, Cycloalkylalkyl- und Aralkylrest bedeutet, wobei die Reste wahlweise mit Halogenresten und Resten mit der Formel -OR⁹ und -SR⁹, worin R⁹ einen Alkylrest bedeutet, substituiert sind.

23. Verwendung nach Anspruch 1, wobei R² CH₃SCH₂CH₂-, einen Isobutyl-, n-Butyl-, Benzyl-, 2-Naphthylmethyl- und Cyclohexylmethylrest bedeutet.

24. Verwendung nach Anspruch 1, wobei R² einen Benzyl-, 4-Fluorbenzyl- und 2-Naphthylmethylrest bedeutet.

25. Verwendung nach Anspruch 1, wobei R³, R⁴ und R⁵ unabhängig voneinander einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- und Heteroaralkylrest bedeuten.

26. Verwendung nach Anspruch 1, wobei R³ einen Benzyl-, p-Fluorbenzyl-, p-Methoxybenzyl-, p-Methylbenzyl- und 2-Naphthylmethylrest und R⁴ einen *tert*.-Butylrest bedeutet.

27. Verwendung nach Anspruch 1, wobei R⁴ und R⁵ zusammen mit dem Stickstoffatom, an welches sie gebunden sind, einen Pyrrolidinyl-, Piperidinyl-, Morpholinyl- und Piperazinylrest bedeuten.

28. Verwendung nach Anspruch 15, wobei die Retrovirusinfektion eine HIV-Infektion ist.

29. Verwendung einer Verbindung mit der Formel 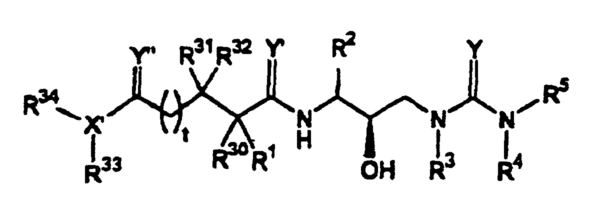 für die Herstellung eines Arzneimittels zur Behandlung von AIDS, worin
- R¹ Wasserstoff, -CH₂SO₂NH₂, einen Alkyl- und Cycloalkylrest und eine Aminosäureseitenkette, die aus der Gruppe ausgewählt ist, die aus Asparagin, S-Methylcystein und deren entsprechenden Sulfoxid- und Sulfonderivaten, einer Glycin-, Alloisoleucin-, Alanin-, Leucin-, *tert*.-Leucin-, Phenylalanin-, Ornithin-, Threonin-, Allothreonin-, Isoleucin-, Histidin-, Norleucin-, Valin-, Glutamin-, Serin-, Asparaginsäure- und β-Cyanoalaninseitenkette besteht,
- R² einen Alkyl-, Aryl-, Cycloalkyl-, Cycloalkylalkyl- und Aralkylrest, der wahlweise mit einer Gruppe substituiert ist, die aus -OR⁹, -SR⁹ und einem Halogenrest ausgewählt ist, wobei R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Heteroaryl-, Aralkyl- und Heteroalkylrest bedeutet und
- R⁴ und R⁵ unabhängig voneinander Wasserstoff und einen wie für R³ definierten Rest oder R⁴ und R⁵ zusammen mit dem Stickstoffatom, an welches sie gebunden sind, einen Heterocycloalkyl- und Heteroarylrest bedeuten,
- R³⁰, R³¹ und R³² jeweils einen wie für R¹ definierten Rest oder einer von R¹ und R³⁰ zusammen mit einem von R³¹ und R³² und den Kohlenstoffatomen, an welche sie gebunden sind, einen Cycloalkylrest bilden, und
- R³³ und R³⁴ unabhängig voneinander Wasserstoff und einen wie für R³ definierten Rest oder R³³ und R³⁴ zusammen mit X' einen Cycloalkyl-, Aryl-, Heterocyclyl- und Heteroarylrest, vorausgesetzt, dass, wenn X' O bedeutet, R³⁴ fehlt, bedeuten,
- X' N, O oder C(R¹⁷) bedeutet, worin R¹⁷ Wasserstoff und einen Alkylrest bedeutet, und Y, Y' und Y" unabhängig voneinander O und S bedeuten und
- t 0 oder 1 bedeutet, worin
• ein Alkylrest einen Alkylrest mit 1 bis 10 Kohlenstoffatomen,
• ein Cycloalkylrest einen Cycloalkylrest mit 3 bis 8 Kohlenstoffatomen,
• ein Aralkylrest einen Alkylrest mit 1 bis 10 Kohlenstoffatomen, in welchem ein Wasserstoffatom durch einen Arylrest ersetzt ist,
• ein Arylrest einen Phenyl- oder Naphthylrest, der wahlweise einen oder mehrere Substituenten trägt, die aus Alkyl, Alkoxy, Halogen, Hydroxy, Amino und Nitro ausgewählt sind,
• ein Heterocycloalkylrest einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, der wahlweise an einem oder mehreren Kohlenstoffatomen mit Halogen, Alkyl, Alkoxy oder Oxo und/oder an einem sekundären Stickstoffatom mit Alkyl, Aralkoxycarbonyl, Alkanoyl, Phenyl oder Phenylalkyl oder an einem tertiären Stickstoffatom mit Oxido substituiert ist und welcher über ein Kohlenstoffatom gebunden ist,
• ein Heteroarylrest einen aromatischen monocyclischen, bicyclischen oder tricyclischen Heterocyclus, der die Heteroatome enthält und wahlweise wie zuvor für den Heterocycloalkylrest definiert substituiert ist, und
• ein Heteroaralkylrest einen Alkylrest mit 1 bis 10 Kohlenstoffatomen, in welchem ein Wasserstoffatom durch einen Heteroarylrest ersetzt ist,
bedeutet.

30. Verwendung nach Anspruch 29, wobei Y, Y' und Y" O bedeuten.

31. Verwendung nach Anspruch 29, wobei t 0 bedeutet.

32. Verwendung nach Anspruch 29, wobei X' N bedeutet.

33. Verwendung nach Anspruch 29, wobei R₁ Wasserstoff und einen Alkylrest mit 1 bis etwa 4 Kohlenstoffatomen, Aralkyl- und Hydroxylrest und einen Rest mit der Formel CH₂C(O)R", worin R" -R³⁸ bedeutet, und -NR³⁸R³⁹, worin R³⁸ und R³⁹ unabhängig voneinander Wasserstoff und einen Alkylrest mit 1 bis etwa 4 Kohlenstoffatomen bedeuten, bedeutet.

34. Verwendung nach Anspruch 33, wobei R¹ einen Wasserstoff-, Methyl-, Ethyl-, Benzyl-, Phenylpropyl-und Hydroxylrest und einen Rest mit der Formel -CH₂C(O)R" bedeutet, worin R" -CH₃, -NH₂ und -OH bedeutet.

35. Verwendung nach Anspruch 29, wobei X' O bedeutet, R³⁴ fehlt und R³³ einen Wasserstoff-, Alkyl- oder Aralkylrest bedeutet.

36. Verwendung nach Anspruch 29, wobei R² einen Alkyl-, Cycloalkylalkyl- und Aralkylrest bedeutet, wobei die Reste wahlweise mit Halogenresten und Resten mit der Formel -OR⁹ und -SR⁹, worin R⁹ einen Alkylrest bedeutet, substituiert sind.

37. Verwendung nach Anspruch 29, wobei R² CH₃SCH₂CH₂-, einen Isobutyl-, n-Butyl-, Benzyl-, 2-Naphthylmethyl- und Cyclohexylmethylrest bedeutet.

38. Verwendung nach Anspruch 29, wobei R² einen Benzyl-, 4-Fluorbenzyl- und 2-Naphthylmethylrest bedeutet.

39. Verwendung nach Anspruch 29, wobei R³, R⁴ und R⁵ unabhängig voneinander einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- und Heteroaralkylrest bedeuten.

40. Verwendung nach Anspruch 29, wobei R³ einen Benzyl-, p-Fluorbenzyl-, p-Methoxybenzyl-, p-Methylbenzyl- und 2-Naphthylmethylrest und R⁴ einen *tert.*-Butylrest bedeutet.

41. Verwendung nach Anspruch 29, wobei R⁴ und R⁵ zusammen mit dem Stickstoffatom, an welches sie gebunden sind, einen Pyrrolidinyl-, Piperidinyl-, Morpholinyl- und Piperazinylrest bedeuten.

42. Verwendung nach einem der Ansprüche 1, 15 und 29, wobei die Verbindung aus der Gruppe ausgewählt ist, die besteht aus Verbindungen mit folgender allgemeiner Formel: 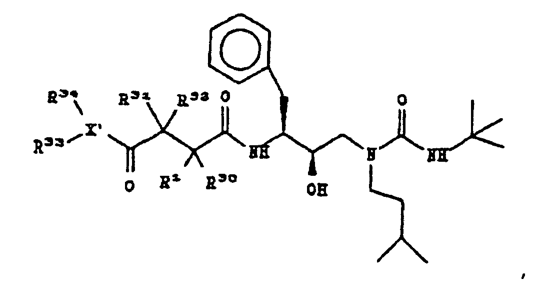 wobei die Substituenten in der folgenden Tabelle aufgeführt sind:
| R¹ | R³⁰ | R³¹ | R³² | X' | R³³ | R³⁴ |
|---|---|---|---|---|---|---|
| H | H | H | H | N | H | H |
| H | H | H | H | O | H | - |
| H | H | H | H | O | CH₃ | - |
| CH₃ | H | H | H | N | H | H |
| CH₃ | H | H | H | O | H | - |
| H | H | CH₃ | H | N | H | H |
| H | H | CH₃ | H | O | H | - |
| CH₃ | CH₃ | H | H | N | H | H |
| CH₃ | CH₃ | H | H | O | H | - |
| CH₃ | CH₃ | H | H | O | CH₂C₆H₄OCH₃ | - |
| H | H | CH₃ | CH₃ | N | H | H |
| H | H | CH₃ | CH₃ | O | H | - |
| H | H | CH₃ | CH₃ | O | CH₂C₆H₄OCH₃ | - |
| CH₃ | H | CH₃ | H | N | H | H |
| CH₃ | H | CH₃ | H | N | H | CH₃ |
| CH₃ | H | CH₃ | H | N | CH₃ | CH₃ |
| CH₃ | H | CH₃ | H | O | H | - |
| CH₃ | H | CH₃ | H | N | H | CH₂C₆H₅OCH₃ |
| OH | H | H | H | N | H | H |
| OH | H | H | H | O | H | - |
| H | H | OH | H | N | H | H |
| H | H | OH | H | O | H | - |
| CH₂ | H | H | H | N | H | H |
| CH₂C(O)NH₂ | H | H | H | N | H | H |
| CH₂C(O)NH₂ | H | H | H | O | H | - |
| CH₂C(O)NH₂ | H | H | H | O | CH₃ | - |
| CH₂Ph | H | H | H | N | H | H |

43. Verwendung nach einem der Ansprüche 1, 15 und 29, wobei die Verbindung aus der Gruppe ausgewählt ist, die besteht aus Verbindungen mit der Formel

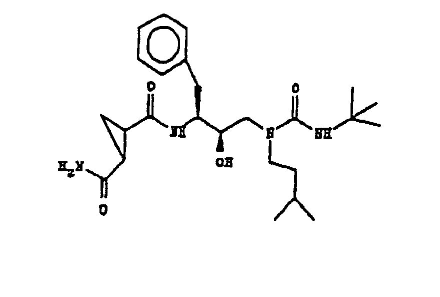

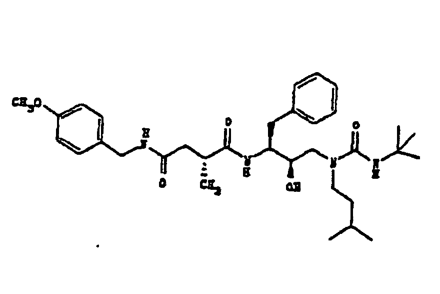

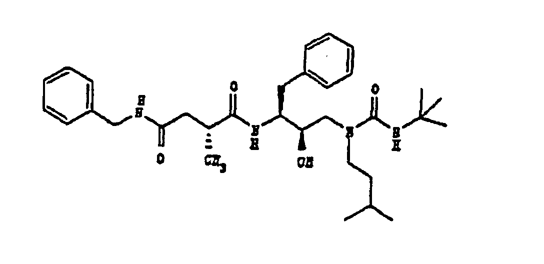

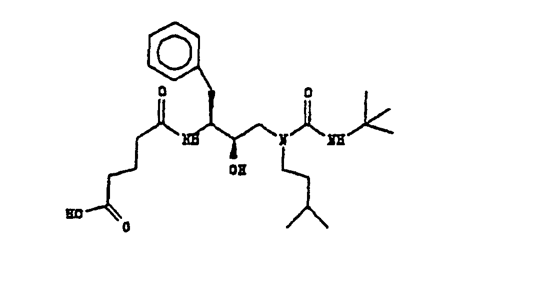

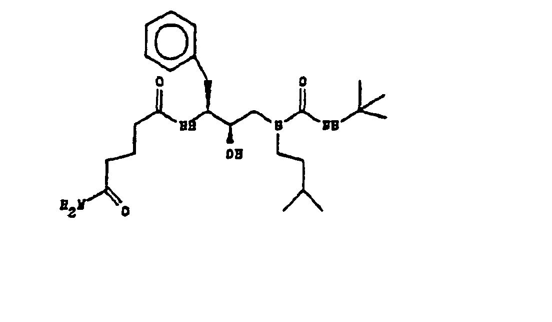

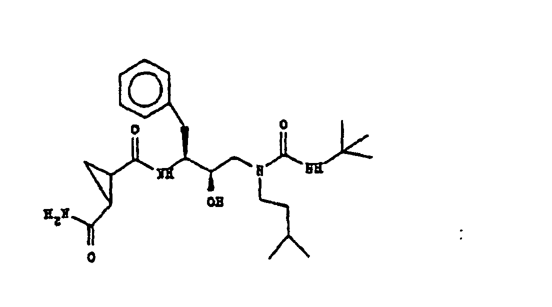

44. Verwendung nach einem der Ansprüche 1, 15 und 29, wobei die Verbindung aus der Gruppe ausgewählt ist, die besteht aus
[1S-[1R*(3S*),2S*]]-4-[[3-[[[1,1-Dimethylethyl)amino]carbonyl]-butylamino-2-hydroxy-1-(phenylmethyl)propyl]amino-2,2,3-trimethyl-4-oxo-butansäure,
[1S-[1R*(3S*),2S*]]-4-[[3-[[[1,1-Dimethylethyl)amino]carbonyl]-(phenylmethyl)aminol-2-hydroxy-1-(phenylmethyl)propyl]-amino-2,2,3-trimethyl-4-oxo-butansäure,
[1S-[1R*(3S*),2S*]]-4-[[3-[[[1,1-Dimethylethyl)amino]carbonyl](4-fluorphenylmethyl)amino]-2-hydroxy-1-(phenylmethyl)propyl]amino-2,2,3-trimethyl-4-oxo-butansäure,
[1S-[1R*(3S*),2S*]]-4-[[3-[[[1,1-Dimethylethyl)amino]carbonyl](cyclohexylmethyl)amino]2-hydroxy-1-(phenylmethyl)propyl]amino-2,2,3-trimethyl-4-oxo-butansäure,
[1S-[1R*(2R*,3S*),2S*]]-N-[3-[[[1,1-Dimethylethyl)amino]carbonyl](3-methylbutyl)amino]-2-hydroxy-1-(phenylmethyl)propyl]-2,3-dimethyl-butandiamid,
[1S-[1R*(3S*),2S*]]-4-[3-[[[1,1-Dimethylethyl)amino]carbonyl](3-methylbutyl)amino]-2-hydroxy-1-(phenylmethyl)propyl]amino-2,2,3-trimethyl-4-oxo-butansäure.

## Revendications

1. Utilisation d'un composé représenté par la formule : 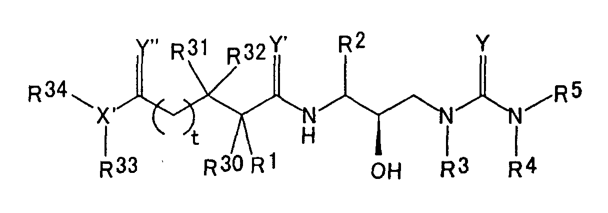 pour préparer un médicament pour inhiber une protéase rétrovirale, dans laquelle :
R¹ représente hydrogène, -CH₂SO₂NH₂, des radicaux alkyle et cycloalkyle et des chaînes latérales d'acide aminé, choisi parmi l'asparagine, la S-méthylcystéine et ses dérivés sulfoxyde et sulfone correspondants, la glycine, l'alloisoleucine, l'alanine, la leucine, la t-leucine, la phénylalanine, l'ornithine, la thréonine, l'allothréonine, l'isoleucine, l'histidine, la norleucine, la valine, la glutamine, la sérine, l'acide aspartique et la bêta-cyanoalanine ;
R² représente des radicaux alkyle, aryle, cycloalkyle, cycloalkylalkyle et aralkyle, le cas échéant substitués par un radical choisi parmi -OR⁹, -SR⁹, et des radicaux halogène, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, hétéroaryle, aralkyle et hétéroaralkyle ;
R⁴ et R⁵ représentent indépendamment, hydrogène et les radicaux tels que définis pour R³, ou R⁴ et R⁵ représentent avec l'atome d'azote auquel ils sont liés, des radicaux hétérocycloalkyle et hétéroaryle ;
R³⁰, R³¹ et R³² représentent les radicaux tels que définis pour R¹, ou un de R¹ et R³⁰ avec un de R³¹ et R³² et les atomes de carbone auxquels ils sont liés, forment un radical cycloalkyle ;
R³³ et R³⁴ représentent indépendamment, hydrogène et les radicaux tels que définis pour R³, ou R³³ et R³⁴ avec X' représentent des radicaux cycloalkyle, aryle, hétérocyclyle et hétéroaryle, pourvu que lorsque X' est O, R³⁴ est absent ;
X' représente N, O ou C(R¹⁷), où R¹⁷ représente hydrogène et des radicaux alkyle ;
Y, Y' et Y'' représentent indépendamment, O et S,
t représente 0 ou 1, et
dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

2. Utilisation selon la revendication 1, où Y, Y' et Y'' sont O.

3. Utilisation selon la revendication 1, où t est 0.

4. Utilisation selon la revendication 1, où X' représente N.

5. Utilisation selon la revendication 1, où R¹ représente hydrogène et des radicaux alkyle ayant de 1 à 4 atomes de carbone, des radicaux aralkyle, le radical hydroxyle et des radicaux représentés par la formule CH₂C(O)R", où R" représente R³⁸, et NR³⁸R³⁹ où R³⁸ et R³⁹ représentent indépendamment, hydrogène et des radicaux alkyle ayant de 1 à 4 atomes de carbone.

6. Utilisation selon la revendication 1, où R¹ représente hydrogène, les radicaux méthyle, éthyle, benzyle, phénylpropyle, hydroxyle et les radicaux représentés par la formule CH₂C(O)R'', où R" représente -CH₃, -NH₂ et -OH.

7. Utilisation selon la revendication 1, où X' représente O, R³⁴ est absent et R³³ représente hydrogène, un radical alkyle ou un radical aralkyle.

8. Utilisation selon la revendication 1, où R² représente des radicaux alkyle, cycloalkylalkyle et aralkyle, lesquels radicaux sont le cas échéant substitués par des radicaux halogène et des radicaux représentés par la formule -OR⁹, -SR⁹, où R⁹ représente des radicaux alkyle

9. Utilisation selon la revendication 1, où R² représente les radicaux CH₃SCH₂CH₂-, isobutyle, n-butyle, benzyle, 2-naphtylméthyle et cyclohexylméthyle.

10. Utilisation selon la revendication 1, où R² représente les radicaux benzyle, 4-fluorobenzyle et 2-aphtylméthyle.

11. Utilisation selon la revendication 1, où R³, R⁴ et R⁵ représentent indépendamment les radicaux alkyle, alcényle, hydroxyalkyle, cycloalkyle, cycloalkylalkyle, hétérocycloalkyle, hétérocycloalkylalkyle, aryle, aralkyle et hétéroaralkyle.

12. Utilisation selon la revendication 1, où R³ représente les radicaux benzyle, para-fluorobenzyle, para-méthoxybenzyle, para-méthylbenzyle et 2-naphtyl-méthyle et R⁴ représente t-butyle.

13. Utilisation selon la revendication 1, où R⁴ et R⁵ représentent avec l'azote auquel ils sont liés, les radicaux pyrrolidinyle, pipéridinyle, morpholinyle et pipérazinyle.

14. Utilisation selon la revendication 1, où la protéase rétrovirale est la protéase de HIV.

15. Utilisation d'un composé représenté par la formule : 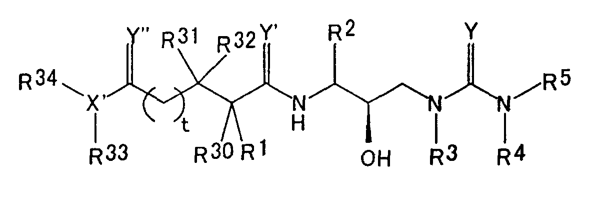 pour préparer un médicament pour traiter une infection rétrovirale, dans laquelle :
R¹ représente hydrogène, -CH₂SO₂NH₂, des radicaux alkyle et cycloalkyle et des chaînes latérales d'acide aminé, choisi parmi l'asparagine, la S-méthylcystéine et ses dérivés sulfoxyde et sulfone correspondants, la glycine, l'alloisoleucine, l'alanine, la leucine, la t-leucine, la phénylalanine, l'ornithine, la thréonine, l'allothréonine, l'isoleucine, l'histidine, la norleucine, la valine, la glutamine, la sérine, l'acide aspartique et la bêta-cyanoalanine ;
R² représente des radicaux alkyle, aryle, cycloalkyle, cycloalkylalkyle et aralkyle, le cas échéant substitués par un radical choisi parmi -OR⁹, -SR⁹, et des radicaux halogène, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, hétéroaryle, aralkyle et hétéroaralkyle ;
R⁴ et R⁵ représentent indépendamment, hydrogène et les radicaux tels que définis pour R³, ou R⁴ et R⁵ représentent avec l'atome d'azote auquel ils sont liés, des radicaux hétérocycloalkyle et hétéroaryle ;
R³⁰, R³¹ et R³² représentent les radicaux tels que définis pour R¹, ou un de R¹ et R³⁰ avec un de R³¹ et R³² et les atomes de carbone auxquels ils sont liés, forment un radical cycloalkyle ;
R³³ et R³⁴ représentent indépendamment, hydrogène et les radicaux tels que définis pour R³, ou R³³ et R³⁴ avec X' représentent des radicaux cycloalkyle, aryle, hétérocyclyle et hétéroaryle, pourvu que lorsque X' est O, R³⁴ est absent ;
X' représente N, O ou C(R¹⁷), où R¹⁷ représente hydrogène et des radicaux alkyle ;
Y, Y' et Y'' représentent indépendamment, 0 et S,
t représente 0 ou 1, et
dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

16. Utilisation selon la revendication 15, où Y, Y' et Y'' sont O.

17. Utilisation selon la revendication 15, où t est 0.

18. Utilisation selon la revendication 15, où X' représente N.

19. Utilisation selon la revendication 15, où R¹ représente hydrogène et des radicaux alkyle ayant de 1 à 4 atomes de carbone, des radicaux aralkyle, le radical hydroxyle et des radicaux représentés par la formule CH₂C(O)R", où R" représente R³⁸, et NR³⁸R³⁹ où R³⁸ et R³⁹ représentent indépendamment, hydrogène et des radicaux alkyle ayant de 1 à 4 atomes de carbone.

20. Utilisation selon la revendication 15, où R¹ représente hydrogène, les radicaux méthyle, éthyle, benzyle, phénylpropyle, hydroxyle et les radicaux représentés par la formule CH₂C(O)R'', où R" représente -CH₃, -NH₂ et -OH.

21. Utilisation selon la revendication 15, où X' représente O, R³⁴ est absent et R³³ représente hydrogène, un radical alkyle ou un radical aralkyle.

22. Utilisation selon la revendication 15, où R² représente des radicaux alkyle, cycloalkylalkyle et aralkyle, lesquels radicaux sont le cas échéant substitués par des radicaux halogène et des radicaux représentés par la formule -OR⁹, -SR⁹, où R⁹ représente des radicaux alkyle

23. Utilisation selon la revendication 15, où R² représente les radicaux CH₃SCH₂CH₂-, isobutyle, n-butyle, benzyle, 2-naphtylméthyle et cyclohexylméthyle.

24. Utilisation selon la revendication 15, où R² représente les radicaux benzyle, 4-fluorobenzyle et 2-aphtylméthyle.

25. Utilisation selon la revendication 15, où R³, R⁴ et R⁵ représentent indépendamment les radicaux alkyle, alcényle, hydroxyalkyle, cycloalkyle, cycloalkylalkyle, hétérocycloalkyle, hétérocycloalkylalkyle, aryle, aralkyle et hétéroaralkyle.

26. Utilisation selon la revendication 15, où R³ représente les radicaux benzyle, para-fluorobenzyle, para-méthoxybenzyle, para-méthylbenzyle et 2-naphtyl-méthyle et R⁴ représente t-butyle.

27. Utilisation selon la revendication 15, où R⁴ et R⁵ représentent avec l'azote auquel ils sont liés, les radicaux pyrrolidinyle, pipéridinyle, morpholinyle et pipérazinyle.

28. Utilisation selon la revendication 15, où l'infection rétrovirale est une infection HIV.

29. Utilisation d'un composé représenté par la formule : 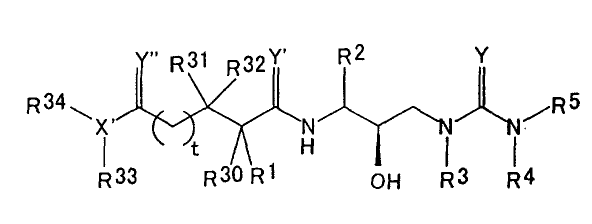 pour préparer un médicament pour traiter le SIDA, dans laquelle :
R¹ représente hydrogène, -CH₂SO₂NH₂, des radicaux alkyle et cycloalkyle et des chaînes latérales d'acide aminé, choisi parmi l'asparagine, la S-méthylcystéine et ses dérivés sulfoxyde et sulfone correspondants, la glycine, l'alloisoleucine, l'alanine, la leucine, la t-leucine, la phénylalanine, l'ornithine, la thréonine, l'allothréonine, l'isoleucine, l'histidine, la norleucine, la valine, la glutamine, la sérine, l'acide aspartique et la bêta-cyanoalanine ;
R² représente des radicaux alkyle, aryle, cycloalkyle, cycloalkylalkyle et aralkyle, le cas échéant substitués par un radical choisi parmi -OR⁹, -SR⁹, et des radicaux halogène, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, hétéroaryle, aralkyle et hétéroaralkyle ;
R⁴ et R⁵ représentent indépendamment, hydrogène et les radicaux tels que définis pour R³, ou R⁴ et R⁵ représentent avec l'atome d'azote auquel ils sont liés, des radicaux hétérocycloalkyle et hétéroaryle ;
R³⁰, R³¹ et R³² représentent les radicaux tels que définis pour R¹, ou un de R¹ et R³⁰ avec un de R³¹ et R³² et les atomes de carbone auxquels ils sont liés, forment un radical cycloalkyle ;
R³³ et R³⁴ représentent indépendamment, hydrogène et les radicaux tels que définis pour R³, ou R³³ et R³⁴ avec X' représentent des radicaux cycloalkyle, aryle, hétérocyclyle et hétéroaryle, pourvu que lorsque X' est O, R³⁴ est absent ;
X' représente N, O ou C(R¹⁷), où R¹⁷ représente hydrogène et des radicaux alkyle ;
Y, Y' et Y'' représentent indépendamment, O et S,
t représente 0 ou 1, et
dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

30. Utilisation selon la revendication 29, où Y, Y' et Y'' sont O.

31. Utilisation selon la revendication 29, où t est 0.

32. Utilisation selon la revendication 29, où X' représente N.

33. Utilisation selon la revendication 29, où R¹ représente hydrogène et des radicaux alkyle ayant de 1 à 4 atomes de carbone, des radicaux aralkyle, le radical hydroxyle et des radicaux représentés par la formule CH₂C(O)R", où R" représente R³⁸, et NR³⁸R³⁹ où R³⁸ et R³⁹ représentent indépendamment, hydrogène et des radicaux alkyle ayant de 1 à 4 atomes de carbone.

34. Utilisation selon la revendication 33, où R¹ représente hydrogène, les radicaux méthyle, éthyle, benzyle, phénylpropyle, hydroxyle et les radicaux représentés par la formule CH₂C(O)R", où R" représente -CH₃, -NH₂ et -OH.

35. Utilisation selon la revendication 29, où X' représente 0, R³⁴ est absent et R³³ représente hydrogène, un radical alkyle ou un radical aralkyle.

36. Utilisation selon la revendication 29, où R² représente des radicaux alkyle, cycloalkylalkyle et aralkyle, lesquels radicaux sont le cas échéant substitués par des radicaux halogène et des radicaux représentés par la formule -OR⁹, -SR⁹, où R⁹ représente des radicaux alkyle

37. Utilisation selon la revendication 29, où R² représente les radicaux CH₃SCH₂CH₂-, isobutyle, n-butyle, benzyle, 2-naphtylméthyle et cyclohexylméthyle.

38. Utilisation selon la revendication 29, où R² représente les radicaux benzyle, 4-fluorobenzyle et 2-aphtylméthyle.

39. Utilisation selon la revendication 29, où R³, R⁴ et R⁵ représentent indépendamment les radicaux alkyle, alcényle, hydroxyalkyle, cycloalkyle, cycloalkylalkyle, hétérocycloalkyle, hétérocycloalkylalkyle, aryle, aralkyle et hétéroaralkyle.

40. Utilisation selon la revendication 29, où R³ représente les radicaux benzyle, para-fluorobenzyle, para-méthoxybenzyle, para-méthylbenzyle et 2-naphtyl-méthyle et R⁴ représente t-butyle.

41. Utilisation selon la revendication 29, où R⁴ et R⁵ représentent avec l'azote auquel ils sont liés, les radicaux pyrrolidinyle, pipéridinyle, morpholinyle et pipérazinyle.

42. Utilisation selon l'une quelconque des revendications 1, 15, 29, où le composé est choisi parmi le groupe consistant en les composés de la formule générale suivante : 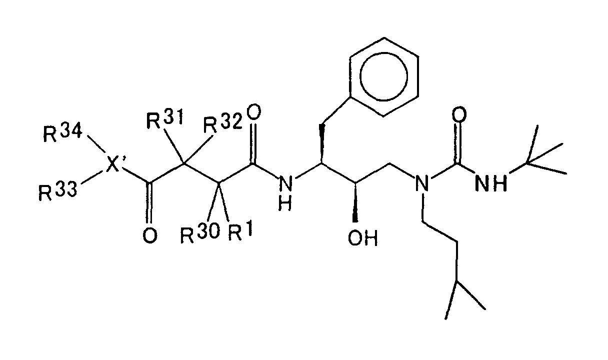 où les substituants sont tels qu'indiqués au tableau suivant :
| R¹ | R³⁰ | R³¹ | R³² | X' | R³³ | R³⁴ |
|---|---|---|---|---|---|---|
| H | H | H | H | N | H | H |
| H | H | H | H | O | H | - |
| H | H | H | H | O | CH₃ | - |
| CH₃ | H | H | H | N | H | H |
| CH₃ | H | H | H | O | H | - |
| H | H | CH₃ | H | N | H | H |
| H | H | CH₃ | H | O | H | - |
| CH₃ | CH₃ | H | H | N | H | H |
| CH₃ | CH₃ | H | H | O | H | - |
| CH₃ | CH₃ | H | H | O | CH₂C₆H₄OCH₃ | - |
| R¹ | R³⁰ | R³¹ | R³² | X' | R³³ | R³⁴ |
| H | H | CH₃ | CH₃ | N | H | H |
| H | H | CH₃ | CH₃ | O | H | - |
| H | H | CH₃ | CH_{3H} | O | CH₂C₆H₄OCH₃ | - |
| CH₃ | H | CH₃ | H | N | H | H |
| CH₃ | H | CH₃ | H | N | H | CH₃ |
| CH₃ | H | CH₃ | H | N | CH_{3H} | CH₃ |
| CH₃ | H | CH₃ | H | O | H | - |
| CH₃ | H | CH₃ | H | N | H | CH₂C₆H₄OCH₃ |
| OH | H | H | H | N | H | H |
| OH | H | H | H | O | H | - |
| H | H | OH | H | N | H | H |
| H | H | OH | H | O | H | - |
| CH₃ | H | H | H | N | H | H |
| CH₂C (O) NH₂ | H | H | H | N | H | H |
| CH₂C(O)NH₂ | H | H | H | O | H | - |
| CH₂C(O)NH₂ | H | H | H | O | CH₃ | - |
| CH₂Ph | H | H | H | N | H | H |

43. Utilisation selon l'une quelconque des revendications 1, 15, 29, où le composé est choisi parmi le groupe consistant en les composés des formules :

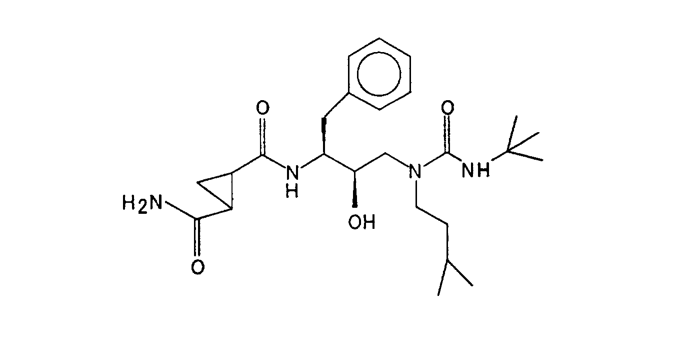

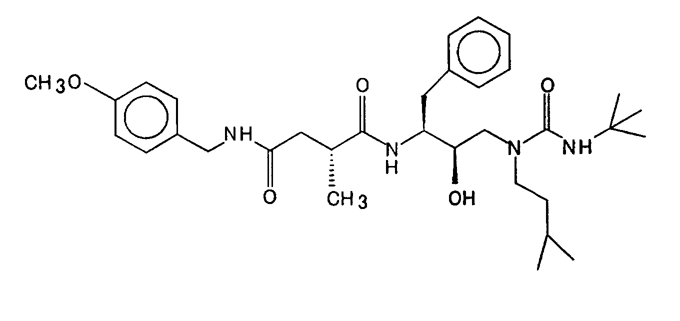

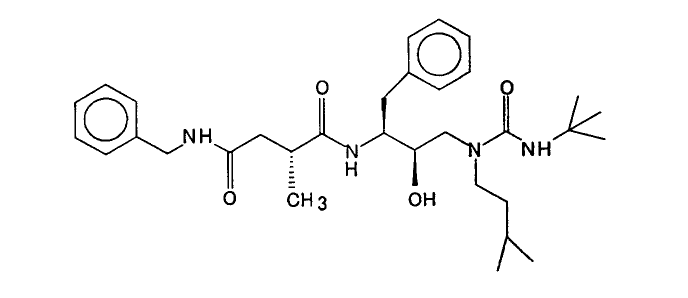

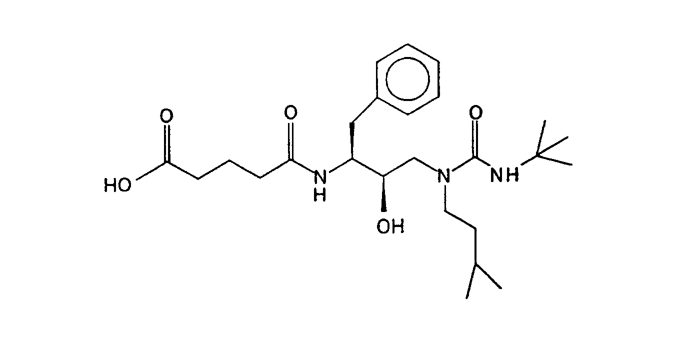

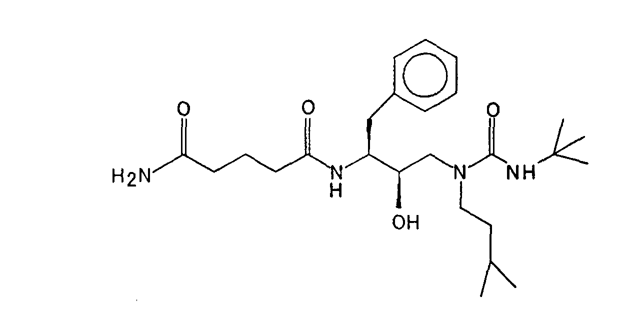

44. Utilisation selon l'une quelconque des revendications 1, 15, 29, où le composé est choisi parmi le groupe consistant en :
l'acide butanoïque, 4-[[3-[[[(1,1-diméthyléthyl)-amino]carbonyl]butylamino]-2-hydroxy-1-(phénylméthyl)-propyl]amino-2,2,3-triméthyl-4-oxo-, [1S-[1R*(3S*),2S*]]-
l'acide butanoïque, 4-[[3-[[[(1,1-diméthyléthyl)-amino]carbonyl](phénylméthyl)amino]-2-hydroxy-1-(phénylméthyl)propyl]amino-2,2,3-triméthyl-4-oxo-, [1S-[1R*(3S*),2S*]]-
l'acide butanoïque, 4-[[3-[[[(1,1-diméthyléthyl)-amino]carbonyl](4-fluorophénylméthyl)amino]-2-hydroxy-1-(phénylméthyl)propyl]amino-2,2,3-triméthyl-4-oxo-, [1S-[1R*(3S*),2S*]]-
l'acide butanoïque, 4-[[3-[[[(1,1-diméthyléthyl)-amino]carbonyl](cyclohexylméthyl)amino]-2-hydroxy-1-(phénylméthyl)propyl]amino-2,2,3-triméthyl-4-oxo-, [1S-[1R*(3S*),2S*]]-
le butanediamide, N-[3-[[[(1,1-diméthyléthyl)-amino]carbonyl](3-méthylbutyl)amino]-2-hydroxy-1-(phénylméthyl)propyl]amino-2,3-diméthyl-, [1S-[1R*(2R*,3S*),2S*]]-
l'acide butanoïque, 4-[3-[[[(1,1-diméthyléthyl)-amino]carbonyl](3-méthylbutyl)amino]-2-hydroxy-1-(phénylméthyl)propyl]amino-2,2,3-triméthyl-4-oxo-, [1S-[1R*(3S*),2S*]]-
